# EUROPEAN PATENT APPLICATION

(11) **EP 2 096 104 A1**
(43) Date of publication of application: **02.09.2009**
(21) Application number: 08169270.9
(22) Date of filing: 29.08.2006
(51) Int. Cl.: C07C 237/04, C07C 237/06, C07D 215/08, C07D 217/06, C07D 217/04, C07D 265/38, C07D 209/86, C07D 233/22, C07D 311/68, C07D 209/42, C07D 307/68, C07D 263/58, C07D 401/04, C07D 319/20, C07D 495/04, C07D 311/58, A61K 31/13, A61K 31/135, A61K 31/137, A61K 31/16, A61K 31/165

(54) **Organic diamines as mGluR7 modulators**

(30) Priority: 31.08.2005 GB 0517740
(62) Divisional of application: 06791701.3
(71) Applicant: NOVARTIS AG, 4002 Basel (CH)
(72) Inventor: Flor, Peter Josef, 4053, Basel (CH); Marzinzik, Andreas, 79576, Weil (DE); Nozulak, Joachim, 79423, Heitersheim (DE); Ofner, Silvio, 4142, Muenchenstein (CH); Roy, Bernard Lucien, 1700, Fribourg (CH); Spanka, Carsten, 79541, Lörrach (DE)
(74) Representative: Vögeli-Lange, Regina

(57) **Abstract**

The invention relates to novel diamines of the formula 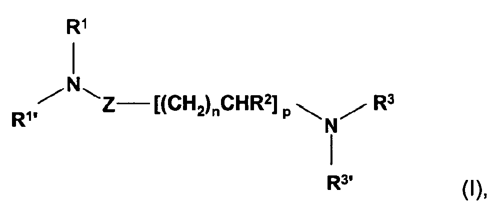 in which all variables are as defined in the specification, in free base form or in acid addition salt form, to their preparation, to their use as medicaments and to medicaments comprising them.

## Description

This invention relates to the use of diamines of formula I for the manufacture of pharmaceutical preparations for the treatment of disorders associated with irregularities of the glutamatergic, GABAergic and/or monoaminergic signal transmission and of nervous system disorders regulated full or in part by mGluR7, novel diamines of formula I, methods of their preparation and pharmaceutical compositions containing them.

Surprisingly, it has been found that the diamines of formula I have advantageous pharmacological properties and are suitable, for example, for the treatment of disorders associated with irregularities of the glutamatergic, GABAergic and/or monoaminergic signal transmission and of nervous system disorders regulated full or in part by mGluR7.

Hence, in a first aspect, the present invention relates to the use of a diamine of formula I 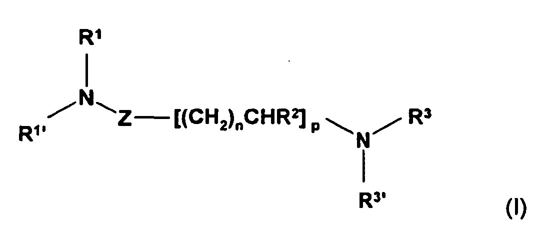 wherein
- Z: is -C(O)-, -C(S)- or a bond;
- n: is 0, 1, 2 or 3;
- p: is 0 or 1, under the proviso that p is 1 when Z is a bond;
- R¹: denotes alkyl which is unsubstituted or mono-, di- or trisubstituted by alkoxy, hydroxy, halogen, amino, alkyl amino, di-alkyl amino, unsubstituted or substituted (C₄-C₁₂)aryloxy, unsubstituted or substituted (C₅-C₁₂)heterocyclyl containing oxygen, sulfur or nitrogen, unsubstituted or substituted (C₃-C₈)cycloalkyl or unsubstituted or substituted (C₄-C₁₄)aryl; alkenyl, which is unsubstituted or mono-, di- or trisubstituted by alkoxy, hydroxy, halogen, amino, alkyl amino, di-alkyl amino, unsubstituted or substituted (C₄-C₁₂)arytoxy, unsubstituted or substituted (C₅-C₁₂)heterocyclyl containing oxygen, sulfur or nitrogen, unsubstituted or substituted (C₃-C₈)cycloalkyl or unsubstituted or substituted (C₄-C₁₂)aryl; unsubstituted or substituted (C₃-C₈)cycloalkyl, unsubstituted or substituted (C₅-C₁₂)heterocydyl containing oxygen, sulfur or nitrogen, unsubstituted or substituted (C₄-C₁₂)aryl or unsubstituted or substituted (C₄-C₁₂)hetaryl; and
- R^{1'}: denotes hydrogen, unsubstituted or substituted (C₄-C₁₂)aryl, unsubstituted or substituted (C₄-C₁₂)aryl alkyl or alkyl which is unsubstituted or mono-, di- or trisubstituted by alkoxy, hydroxy, halogen, amino, alkyl amino or di-alkyl amino; or
- R¹ and R^{1'}: together with the nitrogen atom to which they are attached form an unsubstituted or substituted mono-, di, tri or tetracyclic (C₅-C₁₆)heterocyclyl group containing at least one nitrogen atom,
- R²: denotes hydrogen or alkyl, unsubstituted or substituted (C₄-C₁₂)aryl alkyl or unsubstituted or substituted (C₄-C₁₂)aryl,
- R³: denotes hydrogen, unsubstituted or substituted (C₄-C₁₆)aryl, unsubstituted or substituted (C₃-C₅)cycloalkyl, unsubstituted or substituted (C₅-C₁₂)heterocyclyl containing oxygen, sulfur or nitrogen; unsubstituted or substituted (C₄-C₁₂)hetaryl; alkyl which is unsubstituted or substituted by hydroxy, amino, unsubstituted or substituted (C₄-C₁₂)aryl, unsubstituted or substituted (C₄-C₁₂)hetaryl or unsubstituted or substituted aryl; or R¹⁷-C(O)-, wherein R¹⁷ denotes unsubstituted or substituted alkyl, unsubstituted or substituted (C₄-C₁₂)aryl or unsubstituted or substituted (C₄-C₁₂)hetaryl; and
- R^{3'}: denotes hydrogen, alkyl, alkyl carbonyl or unsubstituted or substituted (C₄-C₁₂)aryl alkyl, or
- R³ and R^{3'}: together with the nitrogen atom to which they are attached form an unsubstituted or substituted cyclic (C₅-C₁₂)heterocyclyl group containing at least one nitrogen atom,
for the manufacture of a medicament for the treatment or prevention of a disorder or condition in which the mGluR7 receptor plays a role or is implicated.

The general terms used hereinbefore and hereinafter preferably have within the context of this disclosure the following meanings, unless otherwise indicated:
Where the plural form is used for compounds, salts, and the like, this is taken to mean also a single compound, salt, or the like.

Alkyl is especially alkyl with from and including 1 up to and including 7, preferably from and including 1 to and including 4, C atoms and is linear or branched; preferably, alkyl is methyl, ethyl, propyl, such as n-propyl or isopropyl, butyl, such as n-butyl, sec-butyl, iso-butyl or tert-butyl.

Alkoxy is especially alkyl with from and including 1 up to and including 7, preferably from and including 1 to and including 4, C atoms and is linear or branched; preferably, alkoxy is methoxy, ethoxy or propoxy, e.g. n-propoxy.

Alkenyl is especially alkenyl with from and including 1 up to and including 7, preferably from and including 1 to and including 4, C atoms and is linear or branched and bound to the molecule via a carbon atom having only single bonds, i.e. which is sp3-hybridized; preferably, alkenyl is allyl.

Halogen or halo is especially fluorine, chlorine, bromine, or iodine, especially fluorine, chlorine, or bromine.

Aryl is a monocyclic, bicyclic or tricyclic aromatic radical having 4 to 14 carbon atoms, in particular 4 to 12, preferably 6 to 12 carbon atoms, being fully unsaturated or partially saturated. In a preferred embodiment, aryl is especially phenyl, fluorenyl, indanyl, acenaphthylenyl, 10, 11-dihydro-5H-dibenzo[a,d]cycloheptenyl, tetrahydronaphthyl or naphthyl.

Heterocyclyl containing oxygen, sulfur or nitrogen as defined herein denotes a mono-, bi-, tri- or tetracyclic heterocyclic system with 1 or 2 heteroatoms especially selected from nitrogen, oxygen,and sulfur, which may be unsaturated or wholly or partly saturated, and is preferably thiazolyl, especially thiazol-5-yl, benzo[b]furyl, especially benzo[b]furan-6-yl, benzoxazolyl, especially benzoxazol-2-yl, pyridyl, especially 2-pyridyl, pyrimidyl, indolyl, especially indol-6-yl, quinolinyl, dihydro-quinolinyl, especially 3,4-dihydro-2*H*-quinolin-1-yl, isoquinolinyl, dihydro-isoquinolinyl, especially 3,4-dihydro-1*H*-isoquinolin-2-yl, phenoxazinyl, especially phenoxazin-10-yl, carbazolyl, especially 1-carbazol-9-yl, dibenzo[b,f]azepinyl, especially dibenzo[b,f]azepin-5-yl, dihydro-dibenzo[b,f]azepinyl, especially 10,11-dihydro-dibenzo[b,f]azepin-5-yl, chromanyl, e.g. 2H-chromanyl, especially 2H-chroman-4-yl, dihydro-chromanyl, e.g. 3,4-dihydro-2H-chromanyl, especially 3,4-dihydro-2H-cbroman-4-yl, piperidinyl, such as piperidin-1-yl or piperidin-4-yl, 5,6,11,12-tetrahydro-dibenzo[a,e]cyclo-octen-5,11-iminyl, especially 5,6,11,12-tetrahydro-dibenzo[a,e]cycloocten-5,11-imine-14-yl, 10,11-dihydro-5*H*-dibenzo[a,d]cyclohepten-5,10-iminyl, benzo[1,4]dioxinyl, e.g. 2,3-dihydro-benzo[1,4]dioxinyl, especially 2,3-dihydro-benzo[1,4]dioxin-2-yl or thieno-pyrimidyl, e.g. thieno[2,3-d]-pyrimidyl, especially thieno[2,3-d]-pyrimidin-4-yl.

Cycloalkyl is especially (C₃-C₈)cycloalkyl, namely cyclopropyl, cyclobutyl, cyclopentyl, cyclohexyl, cycloheptenyl or cyclooctyl, preferably cyclopropyl.

Hetaryl refers to a heterocyclic moiety that is unsaturated in the ring binding the heteroaryl radical to the rest of the molecule in formula I, where at least in the binding ring, but optionally also in any annealed ring, one or more, preferably 1 to 4 carbon atoms are replaced each by a heteroatom selected from the group consisting of nitrogen, oxygen and sulfur; such as thienyl, furyl, pyranyl, thianthrenyl, isobenzofuranyl, benzofuranyl, chromenyl, 2H-pyrrolyl, pyrrolyl, imidazolyl, benzimidazolyl, pyrazolyl, thiazolyl, isothiazolyl, oxazolyl, isoxazolyl, pyridyl, pyrazinyl, pyrimidyl, pyridazyl, indolizinyl, isoindolyl, 3H-indolyl, indolyl, indazolyl, triazolyl, tetrazolyl, purinyl, 4H-quinolizinyl, isoquinolyl, quinolyl, phthalazinyl, naphthyridinyl, quinoxalyl, quinazolinyl, cinnolinyl, pteridinyl, carbazolyl, phenanthridinyl, acridinyl, perimidinyl, phenanthrolinyl, benzo[b]furyl, benzoxazolyl, benzthiazolyl, phenoxazinyl, dibenzo[b,f]azepinyl, chromanyl, benzo[1,4]dioxinyl, or thieno-pyrimidyl. Hetaryl preferably denotes a mono-, bi-, tricyclic heterocyclic system with 1 or 2 heteroatoms consisting of nitrogen, oxygen and sulfur, which is fully unsaturated, and is preferably thiazolyl, especially thiazol-5-yl, benzo[b]furyl, especially benzo[b]furan-6-yl, benzoxazolyl, especially benzoxazol-2-yl, pyridyl, especially 2-pyridyl, pyrimidyl, indolyl, especially indol-6-yl, quinolyl, isoquinolyl, phenoxazinyl, carbazolyl, dibenzo[b,f]azepinyl, chromanyl, e.g. 2H-chromanyl, benzo[1,4]dioxinyl, or thieno-pyrimidyl, e.g. thieno[2,3-d]-pyrimidyl, especially thieno[2,3-d]-pyrimidin-4-yl.

Unless otherwise mentioned, the term "unsubstituted or substituted" as used herein means that the respective radical is unsubstituted or substituted by one or more, preferably up to four, especially one or two substituents, selected from amino, C₁-C₄alkyl amino, di(C₁-C₄alky)-amino, hydroxy-C₁-C₄alkyl amino, phenyl-C₁-C₄alkyl amino, C₃-C₅cycloalkyl amino, di(C₃-C₅)cycloalkyl amino, N-C₁-C₄alkyl-N-C₃-C₅cycloalkyl amino, C₁-C₄alkanoyl amino, halogen, hydroxy, C₁-C₄alkoxy, perfluoro-C₁-C₄alkoxy, C₃-C₆cycloalkyl, C₃-C₅cycloalkyloxy, C₁-C₄alkoxy C₁-C₄alkoxy, di(C₁-C₄alkyl)-amino C₁-C₄alkoxy, carbamoyl, N-C₁-C₄alkylcarbamoyl, N,N-di(C₁-C₄alkyl)-carbamoyl, nitro, cyano, carboxy, C₁-C₄alkoxy carbonyl, C₁-C₄alkanoyl, C₁-C₄alkanoyloxy, benzoyl, amidino, guanidino, ureido, mercapto, C₁-C₄alkylthio, pyridyl, phenyl, phenoxy, phenylthio, phenyl-C₁-C₄alkylthio, C₁-C₄alkylsulfonyl, phenylsulfonyl, C₁-C₄alkylphenylsulfonyl, C₁-C₄alkenyl, C₄-C₈heterocydyl, C₄-C₈heterocydyloxy, C₄-C₈heterocyclyl C₁-C₄alkoxy, C₁-C₄alkylene dioxy bound at adjacent C-atoms of the ring, and C₁-C₄alkyl, which is unsubstituted or substituted by halogen, hydroxy, C₁-C₄alkoxy, nitro, imino, cyano, carboxy, C₁-C₄alkoxy carbonyl, C₁-C₄alkanoyl, C₁-C₄alkanoyloxy or C₄-C₈heterocyclyl.

On account of the asymmetrical carbon atom(s) present in the diamines of formula I and their salts, the compounds may exist in optically active form or in form of mixtures of optical isomers, e.g. in form of racemic mixtures. All optical isomers and their mixtures including the racemic mixtures are part of the present invention.

In view of the close relationship between the novel compounds in free form and those in the form of their salts, including those salts that can be used as intermediates, for example in the purification or identification of the novel compounds, any reference to the free compounds hereinbefore and hereinafter is to be understood as referring also to the corresponding salts, as appropriate and expedient.

Salts are formed, for example, as acid addition salts, preferably with organic or inorganic acids, from compounds of formula I with a basic nitrogen atom, especially the pharmaceutically acceptable salts. For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred.

The compounds of formula I, including their acid addition salts, may also be obtained in the form of hydrates or may include the solvent used for crystallization.

In formula I the following significances are preferred independently, collectively or in any combination or sub-combination:
(a) Z is -C(O)- or a bond;
(b) n is 0, 1 or 2;
(c) p is 0 or 1;
(d) R¹ denotes (C₁₋₆)alkyl, e.g. methyl, which is unsubstituted or mono- or disubstituted by phenyl, which itself is unsubstituted or substituted by halogen, (C₁₋₆)alkoxy or trifluoromethoxy, phenoxy, 2,3-dihydro-benzo[1,4]dioxanyl or (C₅-C₇)cycloalkyl; (C₁₋₆)alkenyl; naphthyl, 9H-fluorenyl, 10,11-dihydro-5H-dibenzo[a,d]cycloheptenyl, 2,3-dihydrobenzo[b]furyl, 2,3-dihydrochromenyl; phenyl which is unsubstituted or substituted by phenyl or (C₁₋₆)alkoxy; or piperidinyl which is substituted by pyrimidyl; and R^{1'} denotes phenyl, hydrogen or (C₁₋₆)alkyl;
(e) alternatively, R¹ and R^{1'} together with the nitrogen atom to which they are attached form a cyclic structure of formuta Ia, Ib, Ic or Id, 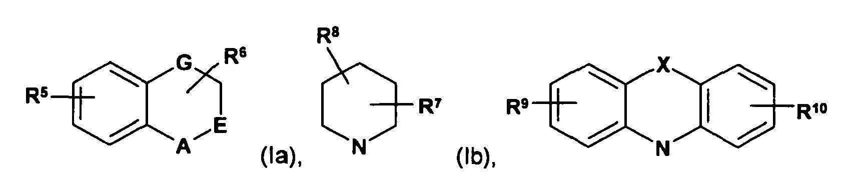 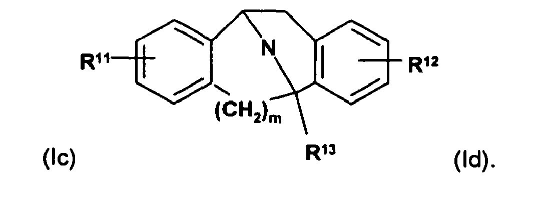
(f) A denotes N and E denotes CR⁴R⁴, or A denotes CR⁴R⁴, and E denotes N,
(g) G denotes CH₂,
(h) X represents oxygen, a bond, -CH=CH- or -CH₂-CH₂-,
(i) m is 0 or 1,
(j) R⁴ and R⁴, represent, independently of each other, hydrogen or (C₁₋₆)alkyl,
(k) R⁵ represents hydrogen, (C₁₋₆)alkyl, CF₃ or halogen,
(l) R⁸ represents hydrogen or (C₁₋₆)alkyl,
(m) R⁷ and R⁸ are both hydrogen,
(n) R⁹ and R¹⁰ are both hydrogen,
(o) R¹¹ is hydrogen or (C₁₋₆)alkoxy,
(p) R¹² represents hydrogen,
(q) R¹³ represents hydrogen or (C₁₋₆)alkyl,
(r) R² denotes hydrogen, (C₁₋₆)alkyl, benzyl, or phenyl, which is unsubstituted or substituted by halogen,
(s) R³ denotes hydrogen, fluorenyl, 2,3-dihydrochromenyl, 1,2,2a,3,4,5-hexahydro-acenaphthyl, 1,2-dihydro-acenaphthyl, tetrahydronaphthyl which is unsubstituted or substituted by (C₁₋₆)alkoxy; benzoxazolyl; cyano-pyridyl; (C₁₋₆)alkyl thieno[2,3-d]-pyrimidyl; N-(C₁₋₆)alkyl piperidinyl, which is substituted by phenyl; (C₃-C₄)cycloalkyl which is substituted by phenyl; 2,3-dihydro-indenyl; (C₁₋₆)alkyl which is unsubstituted or substituted by hydroxy, amino, naphthyl, indolyl, thiazolyl, pyridyl or phenyl, which itself is unsubstituted or mono- or disubstituted by halogen, phenyl, nitro, di-(C₁₋₆)alkyl amino, di-(C₁₋₆)alkyl amino (C₁₋₆)alkoxy, (C₁₋₆)alkyl, (C₁₋₆)alkoxy, trifluoromethyl or trifluoromethoxy; or R¹⁷-C(O)-, wherein R¹⁷ denotes (C₁₋₆)alkyl, phenyl, indolyl or benzo[b]furyl, which in each case is substituted by cyano, carboxy or (C₁₋₆)alkoxy carbonyl;
(t) R³' denotes hydrogen, (C₁₋₆)alkyl, (C₁₋₆)alkyl carbonyl or benzyl,
(u) Alternatively, R³ and R^{3'} together with the nitrogen atom to which they are attached form a cyclic structure of formula Ie or If, 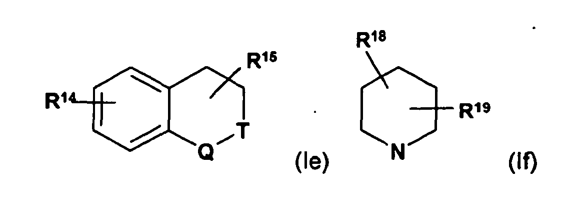
(v) Q denotes N and T denotes CR¹⁶R¹⁶, or Q denotes CR¹⁶R¹⁶, and T denotes N,
(w) R¹⁴ denotes hydrogen,
(x) R¹⁵ denotes hydrogen,
(y) R¹⁸ denotes hydrogen,
   (z1) R¹⁷ denotes hydrogen,
   (z2) R¹⁶ denotes hydrogen,
   (z3) R¹⁸ denotes hydrogen.

In a further aspect, the invention provides processes for the production of the compounds of formula I which comprises the steps as defined below.
a) For the production of a compound of formula I, wherein Z is C(O) and wherein the other radicals and symbols have the meanings as provided for a compound of formula I above, by reacting a halide of formula II 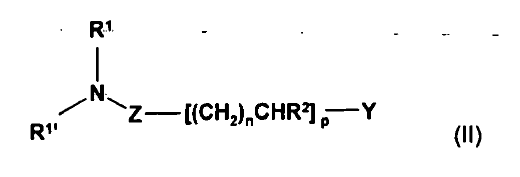 wherein n, p, Z, R¹, R^{1'} and R² are defined as for a compound of formula I above, and Y is CI, Br or I with an amine of formula III 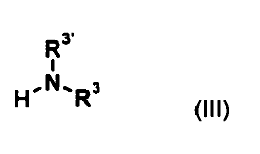 wherein R³ and R³' are defined as for a compound of formula I above.
b) Compounds of formula I, wherein Z is C(O) and wherein the other radicals and symbols have the meanings as provided for a compound of formula I above, can also be prepared by coupling of a carboxylic acid of formula IV 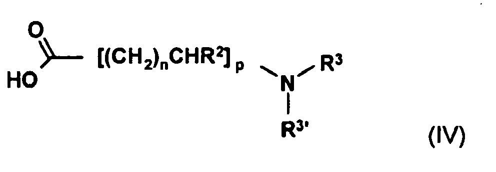 wherein n, p, R³, R^{3'} and R² are as defined above for a compound of formula I, with a primary or secondary amine of formula V 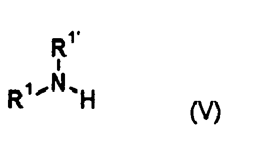 wherein R¹ and R^{1'} are as defined above for a compound of formula I.
c) Alternatively, compounds of formula I, wherein Z is C(O), R³ is R¹⁷-C(O)-, wherein R¹⁷ denotes unsubstituted or substituted alkyl, or R³ is alkyl which is unsubstituted or substituted by hydroxy, amino, unsubstituted or substituted (C₄-C₁₂)aryl, unsubstituted or substituted (C₄-C₁₂)hetaryl or unsubstituted or substituted aryl, R^{3'} is hydrogen and the other radicals and symbols have the meanings as provided for a compound of formula I above, can be synthesized by alkylation, reductive alkylation or acylation of the diamine of formula I, wherein R³ and R^{3'} are both hydrogen, with an alkylating agent, a carbonyl compound or an acylating agent.
d) A compound of formula I, wherein Z is C(O), p is 0 and the other radicals have the meanings as provided for a compound of formula I above, can be prepared by reacting a carbamoyl chloride of formula VI 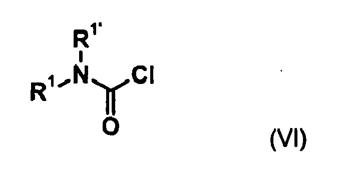 wherein R¹ and R^{1'} defined as above for a compound of formula I, with a primary or secondary amine of formula III 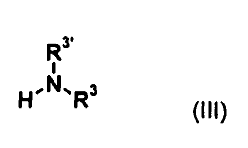 wherein R³ and R^{3'} are defined as above for a compound of formula I.
e) A compound of formula I, wherein Z is a bond and the other radicals have the meanings as provided for a compound of formula I above, can be prepared by subjecting a diamine of formula VII 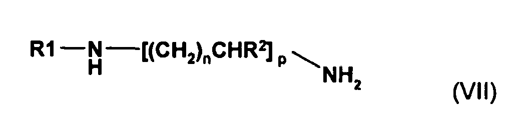
wherein n, p, R¹ and R² are as defined above for a compound of formula I alkylation or acylation known as such to provide diamines of formula I.

The reaction of process a) can be effected according to conventional methods known in the art, e.g. as described in the Examples, e.g. in a suitable solvent such as an alcohol, optionally in the presence of a suitable base, such as potassium carbonate.

The amide formation of process b) can be performed by standard procedures, e.g. as described in the Examples. The reaction can, for instance, be carried out in a suitable solvent in the presence of a base, such as a trialkylamine, optionally a water scavenger, such as dicyclohexyl-carbodiimide or diisopropylcarbodiimide, and, also optionally, hydroxyl-benzotriazol, between room temperature and the reflux temperature of the employed solvent.

Alkylation, reductive alkylation and acylation as described in process c) can be achieved as known in the art. The reductive alkylation can be carried out, e.g., with hydrogenation in the presence of a catalyst, such as platinum or palladium, which is preferably bonded to a carrier material, such as carbon, or a heavy metal catalyst, such as Raney nickel, at normal pressure or at pressures of from 0.1 to 10 MegaPascal (MPa), or with reduction by means of complex hydrides, such as borohydrides, especially alkali metal acetoborohydrides, for example sodium acetoborohydride, in the presence of a suitable acid, preferably relatively weak acids, such as acetic acid, in customary solvents, for example alcohols, such as methanol or ethanol.

Process d) can be carried out between 15 °C and 50 °C, especially about 20 °C to about 25 °C, in a suitable solvent, such as dichloromethane, optionally in the presence of a suitable base, for a duration between 3 and 36 hours, e.g. about 24 hours.

A diamine of formula VII, wherein n, p, R¹ and R² are as defined above for a compound of formula I used as starting material in process e) can be obtained by the following reaction sequence. A compound of formula I, wherein Z is a bond and the other radicals have the meanings as provided for a compound of formula I above, can be prepared by reacting a diamine of formula VIII 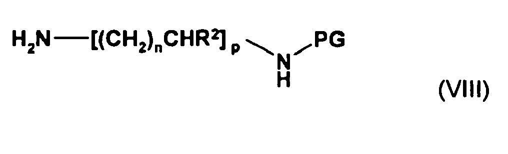 wherein n, p, and R² are as defined above for a compound of formula I and PG represents a protecting group, is reacted with a halogenide of formula IX,

R¹-Hal (IX)

wherein Hal represents halogen, preferably bromo, and R¹ has the meaning as defined above for a compound of formula I to furnish a diamine of formula X, 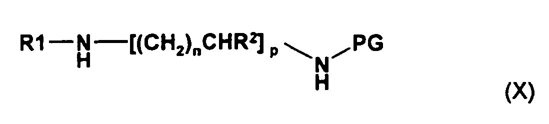 wherein n, p, R¹ and R² are as defined above for a compound of formula I and PG represents a protecting group. Said diamine of formula X is then subjected to reaction conditions under which the protecting group is split off delivering a diamine of formula VII.

All processes a) - d) can also be performed on solid phase using well established methodology, e.g. as described in the Examples.

A so obtained compound of formula I can be converted into another compound of formula I according to conventional methods, e.g. those described in the Examples.

Working up the reaction mixtures according to the above processes and purification of the compounds thus obtained may be carried out in accordance to known procedures.

Acid addition salts may be produced from the free bases in known manner, and vice versa.

Compounds of formula I in optically pure form can be obtained from the corresponding racemates according to well-known procedures. Alternatively, optically pure starting materials can be used.

The starting materials of formulae II - IX are known or may be obtained from known compounds, using conventional procedures, e.g. those described in the Examples.

Compounds of formula I obtained in accordance with the above-described process can also be converted into other compounds of formula I in customary manner, e.g. as described in Examples 114 or 115.

Resulting acid addition salts can be converted into other acid addition salts or into the free bases in a manner known *per* se.

A compound of formula I, wherein Z is C(O), can be converted into the respective compound wherein Z is C(S), for example, by reaction with Lawesson's reagent (2,4-bis-(4-methoxyphenyl)2,4-dithioxo-1,2,3,4-dithiaphosphetan) in a halogenated carbon hydrate, such as dichloromethane, or an aprotic solvent, such as toluene, at temperatures from about 30 °C to reflux.

### Protecting groups

If one or more other functional groups, for example carboxy, hydroxy, amino, or mercapto, are or need to be protected in a compound of formulae II or III, because they should not take part in the reaction, these are such groups as are usually used in the synthesis of peptide compounds, and also of cephalosporins and penicillins, as well as nucleic acid derivatives and sugars.

The protecting groups may already be present in precursors and should protect the functional groups concerned against unwanted secondary reactions, such as acylations, etherifications, esterifications, oxidations, solvolysis, and similar reactions. It is a characteristic of protecting groups that they lend themselves readily, i.e. without undesired secondary reactions, to removal, typically by solvolysis, reduction, photolysis or also by enzyme activity, for example under conditions analogous to physiological conditions, and that they are not present in the end-products. The specialist knows, or can easily establish, which protecting groups are suitable with the reactions mentioned hereinabove and hereinafter.

The protection of such functional groups by such protecting groups, the protecting groups themselves, and their removal reactions are described for example in standard reference works, such as J. F. W. McOmie, "Protective Groups in Organic Chemistry", Plenum Press, London and New York 1973, in T. W. Greene, "Protective Groups in Organic Synthesis", Wiley, New York 1981, in "The Peptides"; Volume 3 (editors: E. Gross and J. Meienhofer), Academic Press, London and New York 1981, in "Methoden der organischen Chemie" (Methods of organic chemistry), Houben Weyl, 4th edition, Volume 15/I, Georg Thieme Verlag, Stuttgart 1974, in H.-D. Jakubke and H. Jescheit, "Aminosäuren, Peptide, Proteine" (Amino acids, peptides, proteins), Verlag Chemie, Weinheim, Deerfield Beach, and Basel 1982, and in Jochen Lehmann, "Chemie der Kohlenhydrate: Monosaccharide und Derivate" (Chemistry of carbohydrates: monosaccharides and derivatives), Georg Thieme Verlag, Stuttgart 1974.

### General process conditions

All process steps described here can be carried out under known reaction conditions, preferably under those specifically mentioned, in the absence of or usually in the presence of solvents or diluents, preferably such as are inert to the reagents used and able to dissolve these, in the absence or presence of catalysts, condensing agents or neutralisiing agents, for example ion exchangers, typically cation exchangers, for example in the H⁺ form, depending on the type of reaction and/or reactants at reduced, normal, or elevated temperature, for example in the range from -100°C to about 190°C, preferably from about -80°C to about 150°C, for example at -80 to -60°C, at room temperature, at - 20 to 40°C or at the boiling point of the solvent used, under atmospheric pressure or in a closed vessel, where appropriate under pressure, and/or in an inert atmosphere, for example under argon or nitrogen.

Salts may be present in all starting compounds and transients, if these contain salt-forming groups. Salts may also be present during the reaction of such compounds, provided the reaction is not thereby disturbed.

The solvents from which those can be selected which are suitable for the reaction in question include for example water, esters, typically lower alkyl-lower alkanoates, e.g diethyl acetate, ethers, typically aliphatic ethers, e.g. diethylether, or cyclic ethers, e.g. tetrahydrofuran, liquid aromatic hydrocarbons, typically benzene or toluene, alcohols, typically methanol, ethanol or 1- or 2-propanol, nitriles, typically acetonitrile, halogenated hydrocarbons, typically dichloromethane, acid amides, typically dimethylformamide, bases, typically heterocyclic nitrogen bases, e.g. pyridine, carboxylic acids, typically lower alkanecarboxylic acids, e.g. acetic acid, carboxylic acid anhydrides, typically lower alkane acid anhydrides, e.g. acetic anhydride, cyclic, linear, or branched hydrocarbons, typically cyclohexane, hexane, or isopentane, or mixtures of these solvents, e.g. aqueous solutions, unless otherwise stated in the description of the process. Such solvent mixtures may also be used in processing, for example through chromatography or distribution.

In a further aspect, the present invention provides novel diamines of formula I, 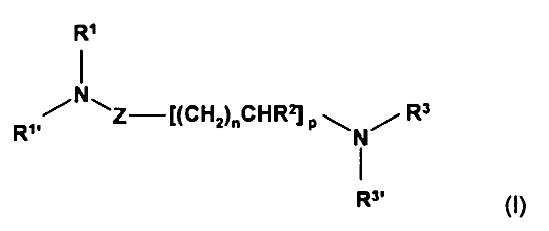 wherein
Z is -C(O)-, -C(S)- or a bond;
n is 0, 1, 2 or 3;
p is 0 or 1, under the proviso that p is 1 when Z is a bond;
R¹ denotes
alkyl which is unsubstituted or mono-, di- or trisubstituted by alkoxy, hydroxy, halogen, amino, alkyl amino, di-alkyl amino, unsubstituted or substituted (C₄-C₁₂)aryloxy, unsubstituted or substituted (C₅-C₁₂)heterocyclyl containing oxygen, sulfur or nitrogen, unsubstituted or substituted (C₃-C₈)cycloalkyl or unsubstituted or substituted (C₄-C₁₄)aryl; alkenyl, which is unsubstituted or mono-, di- or trisubstituted by alkoxy, hydroxy, halogen, amino, alkyl amino, di-alkyl amino, unsubstituted or substituted (C₄-C₁₂)aryloxy, unsubstituted or substituted (C₅-C₁₂)heterocyclyl containing oxygen, sulfur or nitrogen, unsubstituted or substituted (C₃-C₈)cycloalkyl or unsubstituted or substituted (C₄-C₁₂)aryl; unsubstituted or substituted (C₃-C₈)cycloalkyl, unsubstituted or substituted (C₅-C₁₂)heterocyclyl containing oxygen, sulfur or nitrogen, unsubstituted or substituted (C₄-C₁₂)aryl or unsubstituted or substituted (C₄-C₁₂)hetaryl; and
R^{1'} denotes hydrogen, unsubstituted or substituted (C₄-C₁₂)aryl, unsubstituted or substituted (C₄-C₁₂)aryl alkyl or
alkyl which is unsubstituted or mono-, di- or trisubstituted by alkoxy, hydroxy, halogen, amino, alkyl amino or di-alkyl amino; or
R¹ and R^{1'} together with the nitrogen atom to which they are attached form an unsubstituted or substituted mono-, di, tri or tetracyclic (C₅-C₁₆)heterocyclyl group containing at least one nitrogen atom,
R² denotes hydrogen or alkyl, unsubstituted or substituted (C₄-C₁₂)aryl alkyl or unsubstituted or substituted (C₄-C₁₂)aryl,
R³ denotes hydrogen, unsubstituted or substituted (C₄-C₁₆)aryl, unsubstituted or substituted (C₃-C₅)cycloalkyl, (C₅-C₁₂)heterocyclyl containing oxygen, sulfur or nitrogen; unsubstituted or substituted (C₄-C₁₂)hetaryl; alkyl which is unsubstituted or substituted by hydroxy, amino, (C₄-C₁₂)aryl, (C₄-C₁₂)hetaryl or unsubstituted or substituted aryl; or R¹⁷-C(O)-, wherein R¹⁷ denotes alkyl, (C₄-C₁₂)aryl or (C₄-C₁₂)hetaryl; and
R^{3'} denotes hydrogen, alkyl, alkyl carbonyl or unsubstituted or substituted (C₄-C₁₂)aryl alkyl, or
R³ and R^{3'} together with the nitrogen atom to which they are attached form an unsubstituted or substituted cyclic (C₅-C₁₂)heterocyclyl group containing at least one nitrogen atom,
under the proviso that if n is 0, Z is -C(O)-, p is 1, R¹ is benzyl, R² is phenyl and R^{1'} and R^{3'} are hydrogen, then R³ does not denote benzyl or acetyl; if n is 0, Z is -C(O)-, p is 1, R' is phenethyl, R^{1'} is hydrogen, R² is phenyl and R^{3'} is hydrogen, then R³ does not denote hydrogen; if n is 1, Z is a bond, p is 1, R¹ is di-phenyl methyl and R^{1'}, R² and R^{3'} are all hydrogen, then R³ is not di-phenyl methyl or benzyl; and, if Z is a bond, p is 1, R¹ and R^{1'} together represent the structure of subformula Ic, X represents -CH₂-CH₂-, R² is hydrogen and n is 1 or 2, then R³ and R^{3'} together with the nitrogen atom to which they are attached do not represent piperidinyl and R³ and R^{3'} are not simultaneously methyl; in free base or acid addition salt form.

According to the present invention, such diamines of formula I are preferred, wherein
Z is -C(O)- or a bond;
n is 0, 1 or 2;
p is 0 or 1, under the proviso that p is 1 when Z is a bond;
R¹ denotes
(C₁₋₆)alkyl which is unsubstituted or mono-, di- or trisubstituted by (C₁₋₆)alkoxy, hydroxy, halogen, amino, (C₁₋₆)alkyl amino, di-(C₁₋₆)alkyl amino, aryloxy, 2,3-dihydro-benzo[1,4]dioxanyl, (C₄-C₇)cycloalkyl or phenyl, which itself is unsubstituted or substituted by halogen, (C₁₋₆)alkoxy or trifluoromethoxy;
(C₁₋₆)alkenyl, which is unsubstituted or mono-, di- or trisubstituted by (C₁₋₆)alkoxy, hydroxy, halogen, amino, (C₁₋₆)alkyl amino, di-(C₁₋₆)alkyl amino, aryloxy, 2,3-dihydro-benzo[1,4]dioxanyl, (C₄-C₇)cycloalkyl or phenyl, which itself is unsubstituted or substituted by halogen, (C₁₋₆)alkoxy or trifluoromethoxy;
(C₄-C₇)cycloalkyl, 1,4-dioxan, naphthyl, 9H-fluorenyl, 10,11-dihydro-5H-dibenzo[a,d]cycloheptenyl, 2,3-dihydrobenzo[b]furyl, 2,3-dihydrochromenyl;
phenyl which is unsubstituted or substituted by phenyl or (C₁₋₆)alkoxy; or
piperidinyl which is unsubstituted or substituted by phenyl or pyrimidyl; and
R^{1'} denotes hydrogen, phenyl, benzyl or
(C₁₋₆)alkyl which is unsubstituted or mono-, di- or trisubstituted by (C₁₋₆)alkoxy, hydroxy, halogen, amino, (C₁₋₆)alkyl amino or di-(C₁₋₆)alkyl amino; or
R¹ and R^{1'} together with the nitrogen atom to which they are attached form a cyclic structure of formula Ia, Ib, Ic or Id, 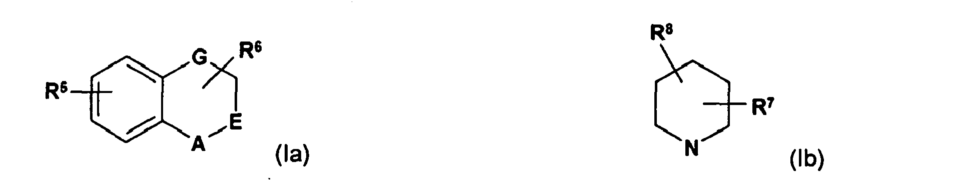 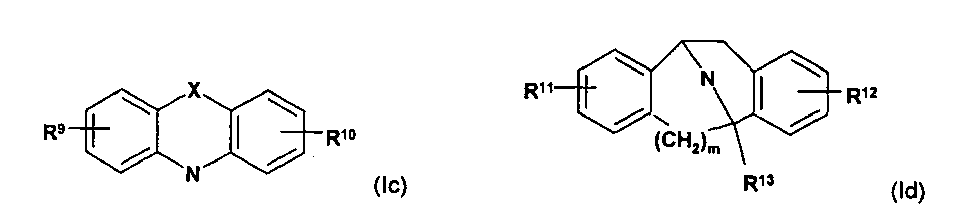 wherein
A denotes N and E denotes CR⁴R⁴, or A denotes CR⁴R⁴, and E denotes N,
G denotes a single bond, CHR¹⁹, -CH₂CH₂-, -CH=CH-, S(O)ₜ or NR²⁰, wherein t is 0, 1 or 2, and R¹⁹ and R²⁰, independently of each other, represent hydrogen or (C₁₋₆)alkyl, which is unsubstituted or mono-, di- or trisubstituted by (C₁₋₆)alkoxy, hydroxy, halogen, amino, (C₁₋₆)alkyl amino or di-(C₁₋₆)alkyl amino
X represents oxygen, a single bond, CHR²¹, -CH=CH- or -CH₂-CH₂-, S(O)ᵣ or NR²², wherein r is 0, 1 or 2, and R²¹ and R²², independently of each other, represent hydrogen or (C₁₋₆)alkyl, which is unsubstituted or mono-, di- or trisubstituted by (C₁₋₆)alkoxy, hydroxy, halogen, amino, (C₁₋₆)alkyl amino or di-(C₁₋₆)alkyl amino,
m is 0 or 1,
R⁴ and R^{4'} represent, independently of each other, hydrogen or (C₁₋₆)alkyl, which is unsubstituted or mono-, di- or trisubstituted by (C₁₋₆)alkoxy, hydroxy, halogen, amino, (C₁₋₆)alkyl amino or di-(C₁₋₆)alkyl amino,
R⁵ represents hydrogen, halogen, (C₁₋₆)alkoxy, phenyl or (C₁₋₆)alkyl which is unsubstituted or mono-, di- or trisubstituted by (C₁₋₆)alkoxy, hydroxy, halogen, amino, (C₁₋₆)alkyl amino or di-(C₁₋₆)alkyl amino,
R⁶ represents hydrogen, halogen, phenyl or (C₁₋₆)alkyl which is unsubstituted or mono-, di- or trisubstituted by (C₁₋₆)alkoxy, hydroxy, halogen, amino, (C₁₋₆)alkyl amino or di-(C₁₋₆)alkyl amino,
R⁷ and R⁸ represent, independently of each other, hydrogen, halogen or (C₁₋₆)alkyl, which is unsubstituted or mono-, di- or trisubstituted by (C₁₋₆)alkoxy, hydroxy, halogen, amino, (C₁₋₆)alkyl amino or di-(C₁₋₆)alkyl amino,
R⁹ and R¹⁰ represent, independently of each other, hydrogen, halogen, (C₁₋₆)alkoxy, phenyl or (C₁₋₆)alkyl which is unsubstituted or mono-, di- or trisubstituted by (C₁₋₆)alkoxy, hydroxy, halogen, amino, (C₁₋₆)alkyl amino or di-(C₁₋₆)alkyl amino,
R¹¹ is hydrogen, halogen, (C₁₋₆)alkoxy, phenyl or (C₁₋₆)alkyl which is unsubstituted or mono-, di- or trisubstituted by (C₁₋₆)alkoxy, hydroxy, halogen, amino, (C₁₋₆)alkyl amino or di-(C₁₋₆)alkyl amino,
R¹² is hydrogen, halogen, (C₁₋₆)alkoxy, phenyl or (C₁₋₆)alkyl which is unsubstituted or mono-, di- or trisubstituted by (C₁₋₆)alkoxy, hydroxy, halogen, amino, (C₁₋₆)alkyl amino or di-(C₁₋₆)alkyl amino, and
R¹³ represents hydrogen or (C₁₋₆)alkyl which is unsubstituted or mono-, di- or trisubstituted by (C₁₋₆)alkoxy, hydroxy, halogen, amino, (C₁₋₆)alkyl amino or di-(C₁₋₆)alkyl amino,
R² denotes hydrogen or (C₁₋₆)alkyl, or
benzyl, naphthyl or phenyl, which in each case is unsubstituted or substituted by halogen, (C₁₋₆)alkoxy, (C₁₋₆)alkyl, hydroxy, trifluoromethyl, amino, (C₁₋₆)alkyl amino or di-(C₁₋₆)alkyl amino,
R³ denotes hydrogen, fluorenyl, 2,3-dihydrochromenyl, 1,2,2a,3,4,5-hexahydro-acenaphthyl, 1,2-dihydro-acenaphthyl, tetrahydronaphthyl which is unsubstituted or substituted by (C₁₋₆)alkoxy; benzoxazolyl; cyano-pyridyl; (C₁₋₆)alkyl thieno[2,3-d]-pyrimidyl; N-(C₁₋₆)alkyl piperidinyl, which is substituted by phenyl; (C₃-C₄)cycloalkyl which is substituted by phenyl; 2,3-dihydro-indenyl; (C₁₋₆)alkyl which is unsubstituted or substituted by hydroxy, amino, naphthyl, indolyl, thiazolyl, pyridyl or phenyl, which itself is unsubstituted or mono- or disubstituted by halogen, phenyl, nitro, di-(C₁₋₆)alkyl amino, di-(C₁₋₆)alkyl amino (C₁₋₆)alkoxy,
(C₁₋₆)alkyl, (C₁₋₆)alkoxy, trifluoromethyl or trifluoromethoxy; or R¹⁷-C(O)-, wherein R¹⁷ denotes (C₁₋₆)alkyl, phenyl, indolyl or benzo[b]furyl, which in each case is substituted by cyano, carboxy or (C₁₋₆)alkoxy carbonyl; and
R^{3'} denotes hydrogen, (C₁₋₆)alkyl, (C₁₋₆)alkyl carbonyl or benzyl, or
R³ and R^{3'} together with the nitrogen atom to which they are attached form a cyclic structure of formula Ie or If, 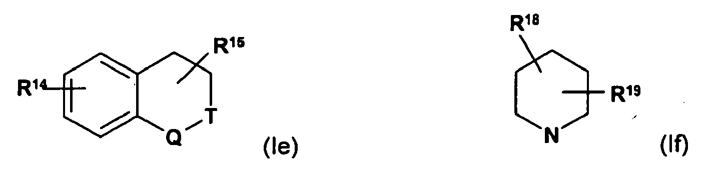 wherein Q denotes N and T denotes CR¹⁶R^{16'} or Q denotes CR¹⁶R^{16'} and T denotes N,
R¹⁴ denotes hydrogen, halogen, (C₁₋₆)alkoxy, phenyl or (C₁₋₆)alkyl which is unsubstituted or mono-, di- or trisubstituted by (C₁₋₆)alkoxy, hydroxy, halogen, amino, (C₁₋₆)alkyl amino or di-
(C₁₋₆)alkyl amino,
R¹⁵, R¹⁶, R^{16'} R¹⁷ and R¹⁸ represent, independently of each other, hydrogen, halogen or (C₁₋₆)alkyl, which is unsubstituted or mono-, di- or trisubstituted by (C₁₋₆)alkoxy, hydroxy, halogen, amino, (C₁₋₆)alkyl amino or di-(C₁₋₆)alkyl amino, under the proviso that if n is 0, Z is -C(O)-, p is 1, R¹ is benzyl, R² is phenyl and R^{1'} and R^{3'} are hydrogen, then R³ does not denote benzyl or acetyl; if n is 0, Z is -C(O)-, p is 1. R¹ is phenethyl, R^{1'} is hydrogen, R² is phenyl and R³, is hydrogen, then R³ does not denote hydrogen; if n is 1, Z is a bond, p is 1, R¹ is di-phenyl methyl and R^{1'}, R² and R^{3'} are all hydrogen, then R³ is not di-phenyl methyl or benzyl; and, if Z is a bond, p is 1, R¹ and R^{1'} together represent the structure of subformula Ic, X represents -CH₂-CH₂-, R² is hydrogen and n is 1 or 2, then R³ and R^{3'} together with the nitrogen atom to which they are attached do not represent piperidinyl and R³ and R^{3'} are not simultaneously methyl.

More preferred are those diamines of formula I, wherein
Z is -C(O)- or a bond;
n is 0, 1 or 2;
p is 0 or 1, under the proviso that p is 1 when Z is a bond;
R¹ denotes
(C₁₋₆)alkyl which is unsubstituted or mono- or disubstituted by phenyl, which itself is unsubstituted or substituted by halogen, (C₁₋₆)alkoxy or trifluoromethoxy, phenoxy, 2,3-dihydro-benzo[1,4]dioxanyl or (C₅-C₇)cycloalkyl;
(C₁₋₆)alkenyl; naphthyl, 9H-fluorenyl, 10.11-dihydro-5H-dibenzo[a,d]cycloheptenyl, 2,3-dihydrobenzo[b]furyl, 2,3-dihydrochromenyl;
phenyl which is unsubstituted or substituted by phenyl or (C₁₋₆)alkoxy; or
piperidinyl which is substituted by pyrimidyl; and
R^{1'} denotes phenyl, hydrogen or (C₁₋₆)alkyl; or
R¹ and R^{1'} together with the nitrogen atom to which they are attached form a cyclic structure of formula Ia, Ib, Ic or Id, 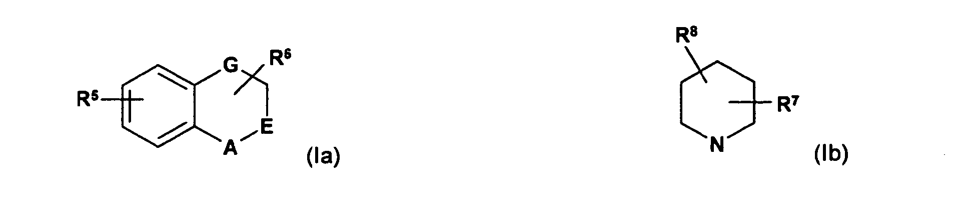 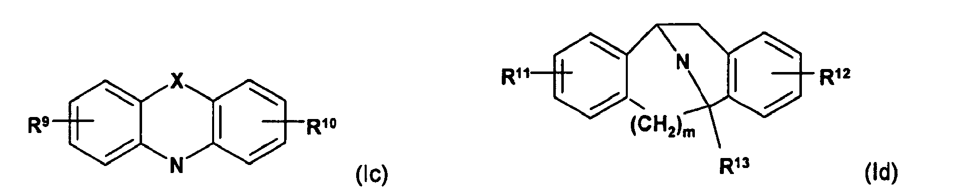 wherein
A denotes N and E denotes CR⁴R⁴, or A denotes CR⁴R⁴, and E denotes N,
G denotes CH₂,
X represents oxygen, a bond, -CH=CH- or -CH₂-CH₂-,
m is 0 or 1,
R⁴ and R^{4'} represent, independently of each other, hydrogen or (C₁₋₆)alkyl,
R⁵ represents hydrogen, (C₁₋₆)alkyl, CF₃ or halogen,
R⁶ represents hydrogen or (C₁₋₆)alkyl,
R⁷ and R⁸ are both hydrogen,
R⁹ and R¹⁰ are both hydrogen,
R¹¹ is hydrogen or (C₁₋₆)alkoxy,
R¹² represents hydrogen, and
R¹³ represents hydrogen or (C₁₋₆)alkyl,
R² denotes hydrogen, (C₁₋₆)alkyl, benzyl, or phenyl, which is unsubstituted or substituted by halogen,
R³ denotes hydrogen, fluorenyl, 2,3-dihydrochromenyl, 1,2,2a,3,4,5-hexahydro-acenaphthyl, 1,2-dihydro-acenaphthyl, tetrahydronaphthyl which is unsubstituted or substituted by (C₁₋₈)alkoxy; benzoxazolyl; cyano-pyridyl; (C₁₋₆)alkyl thieno[2,3-d]-pyrimidyl; N-(C₁₋₆)alkyl
piperidinyl, which is substituted by phenyl; (C₃-C₄)cycloalkyl which is substituted by phenyl; 2,3-dihydro-indenyl; (C₁₋₆)alkyl which is unsubstituted or substituted by hydroxy, amino,
naphthyl, indolyl, thiazolyl, pyridyl or phenyl, which itself is unsubstituted or mono- or disubstituted by halogen, phenyl, nitro, di-(C₁₋₆)alkyl amino, di-(C₁₋₆)alkyl amino (C₁₋₆)alkoxy,
(C₁₋₆)alkyl, (C₁₋₆)alkoxy, trifluoromethyl or trifluoromethoxy; or R¹⁷-C(O)-, wherein R¹⁷ denotes (C₁₋₆)alkyl, phenyl, indolyl or benzo[b]furyl, which in each case is substituted by cyano,
carboxy or (C₁₋₆)alkoxy carbonyl; and
R^{3'} denotes hydrogen, (C₁₋₆)alkyl, (C₁₋₆)alkyl carbonyl or benzyl, or
R³ and R^{3'} together with the nitrogen atom to which they are attached form a cyclic structure of formula le or If, 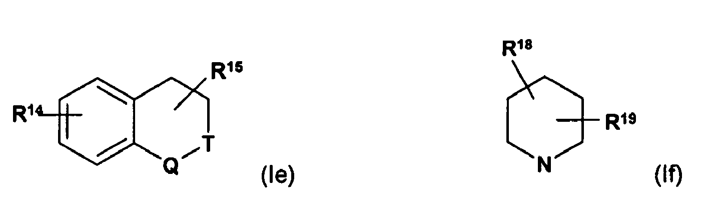 wherein
Q denotes N and T denotes CR¹⁶R¹⁶, or Q denotes CR¹⁶R^{16,} and T denotes N, and
R¹⁴, R¹⁵, R¹⁶, R¹⁶' R¹⁷ and R¹⁸ are all hydrogen, under the proviso that if n is 0, Z is -C(O)-, p is 1, R¹ is benzyl, R² is phenyl and R^{1'} and R^{3'} are hydrogen, then R³ does not denote benzyl or acetyl; if n is 0, Z is -C(O)-, p is 1, R¹ is phenethyl, R^{1'} is hydrogen, R² is phenyl and R^{3,} is hydrogen, then R³ does not denote hydrogen; if n is 1, Z is a bond, p is 1, R¹ is di-phenyl methyl and R^{1'}, R² and R^{3'} are all hydrogen, then R³ is not di-phenyl methyl or benzyl; and, if Z is a bond, p is 1, R¹ and R^{1'} together represent the structure of subformula Ic, X represents -CH₂-CH₂-, R² is hydrogen and n is 1 or 2, then R³ and R^{3'} together with the nitrogen atom to which they are attached do not represent piperidinyl and R³ and R^{3'} are not simultaneously methyl.

Diamines of formula I and their pharmaceutically acceptable acid addition salts, hereinafter referred to as agents of the invention, exhibit valuable pharmacological properties and are therefore useful as pharmaceuticals.

In particular, the agents of the invention exhibit a marked and selective activatory action at human metabotropic glutamate receptors (mGluRs). This can be determined in vitro for example at recombinant human metabotropic glutamate receptors, especially adenylate cyclase-coupled subtypes thereof such as mGluR7, using different procedures like, for example, measurement of inhibition of forskolin-stimulated cAMP accumulation as described by P. J. Flor et. al., Neuropharmacology Vol. 36, pages 153-159 (1997) and F. Gasparini et al., J. Pharmacol. Exp. Ther. Vol 290, pages 1678-1687 (1999) or by determination to what extent the sub-maximal agonist-induced elevation of GTP-gamma-S binding is enhanced as described by M. Maj et al., Neuropharmacology Vol. 45, 895-906 (2003), and references cited therein. Isolation and expression of human mGluR7 subtypes are described in P. J. Flor et. al., Neuropharmacology Vol. 36, pages 153-159 (1997). Selected agents of the invention show EC₅₀ values for the stimulation of GTP-gamma-S binding, sub-maximally induced by DL-AP4, measured on membranes from recombinant cells expressing human mGluR7b or rat mGluR7a of about 1 nM to about 50 µM. Also, selected agents of the invention show EC₅₀ values for the inhibition of forskolin-stimulated cAMP accumulation on recombinant cells expressing human mGluR7b or rat mGluR7a of about 1 nM to about 50 µM.

The agents of the invention are therefore useful in the treatment of disorders associated with irregularities of the glutamatergic, GABAergic and/or monoaminergic signal transmission, and of nervous system disorders regulated full or in part by mGluR7.

Disorders associated with irregularities of the glutamatergic, GABAergic and/or monoaminergic signal transmission are, for example, epilepsy, cerebral ischemias, especially acute ischemias, eye disorders, especially glaucoma and ischemic diseases of the eye, itch, muscle spasms such as local or general spasticity and, in particular, convulsions or pain.

Nervous system disorders regulated full or in part by mGluR7 are for example acute, traumatic and chronic degenerative processes of the nervous system, such as Parkinson's disease, dementia associated with Parkinson's Disease, senile dementia, Alzheimer's disease, Huntington's chorea, amyotrophic lateral sclerosis and multiple sclerosis, psychiatric diseases such as schizophrenia, anxiety disorders and depression.

The usefulness of the agents of the invention in the treatment of the above-mentioned disorders can be confirmed in a range of standard tests including, as an example, the one indicated below:
Activity of the agents of the invention in anxiety can be demonstrated in standard models such as the stress-induced hyperthermia in mice [cf. A. Lecci et al., Psychopharmacol. Vol. 101, pages 255-261 (1990)]. At doses of about 0.1 to about 100 mg/kg p.o. or i.p., the agents of the invention reverse the stress-induced hyperthermia.

For all the above mentioned indications, the appropriate dosage will of course vary depending upon, for example, the compound employed, the host, the mode of administration and the nature and severity of the condition being treated. However, in general, satisfactory results in animals are indicated to be obtained at a daily dosage of from about 0.5 to about 100 mg/kg animal body weight. In larger mammals, for example humans, an indicated daily dosage is in the range from about 5 to 1500 mg, preferably about 10 to about 1000 mg of the compound conveniently administered in divided doses up to 4 times a day.

In accordance with the foregoing, the present invention also provides an agent of the invention for use as a pharmaceutical, e.g. in the treatment of disorders associated with irregularities of the glutamatergic, GABAergic and/or monoaminergic signal transmission, and of nervous system disorders regulated full or in part by mGluR7.

The invention also provides the use of an agent of the invention, in the treatment of disorders associated with irregularities of the glutamatergic, GABAergic and/or monoaminergic signal transmission, and of nervous system disorders regulated full or in part by mGluR7.

Furthermore the invention provides the use of an agent of the invention for the manufacture of a pharmaceutical composition designed for the treatment of disorders associated with irregularities of the glutamatergic, GABAergic and/or monoaminergic signal transmission, and of nervous system disorders regulated full or in part by mGluR7.

In a further aspect the invention relates to a method of treating disorders regulated full or in part by mGluR7, which method comprises administering to a warm-blooded organism in need of such treatment a therapeutically effective amount of an agent of the invention.

The pharmaceutical compositions according to the invention are compositions for enteral, such as nasal, rectal or oral, or parenteral, such as intramuscular or intravenous, administration to warm-blooded animals (human beings and animals) that comprise an effective dose of the pharmacological active ingredient alone or together with a significant amount of a pharmaceutically acceptable carrier. The dose of the active ingredient depends on the species of warm-blooded animal, body weight, age and individual condition, individual pharmacokinetic data, the disease to be treated and the mode of administration.

The pharmaceutical compositions comprise from approximately 1% to approximately 95%, preferably from approximately 20% to approximately 90%, active ingredient. Pharmaceutical compositions according to the invention may be, for example, in unit dose form, such as in the form of ampoules, vials, suppositories, dragées, tablets or capsules.

The pharmaceutical compositions of the present invention are prepared in a manner known *per se,* for example by means of conventional dissolving, lyophilizing, mixing, granulating or confectioning processes.

A compound of formula I can be administered alone or in combination with one or more other therapeutic agents, possible combination therapy taking the form of fixed combinations or the administration of a compound of the invention and one or more other therapeutic agents being staggered or given independently of one another, or the combined administration of fixed combinations and one or more other therapeutic agents.

For instance, if the disorder to be treated is epilepsy, the other therapeutic agents can be selected from barbiturates and derivatives thereof, benzodiazepines, carboxamides, hydantoins, succinimides, valproic acid and other fatty acid derivates, AMPA antagonists and other anti-epileptic drugs. The term "barbiturates and derivatives thereof" as used herein includes, but is not limited to Phenobarbital and primidon. The term "benzodiazepines" as used herein includes, but is not limited to clonazepam, diazepam and lorazepam. The term "carboxamides" as used herein includes, but is not limited to carbamazepine and oxcarbazepine. The term "hydantoins" as used herein includes, but is not limited to phenytoin. The term "succinimides" as used herein includes, but is not limited to ethosuximide, phensuximide and mesuximide. The term "valproic acid and other fatty acid derivates" as used herein includes, but is not limited to valproic acid sodium salt, tiagabine hydrochloride monohydrate and vigrabatrine. The term "other anti-epileptic drugs" as used herein includes, but is not limited to levetiracetam, lamotrigine, gabapentin, sultiam and felbamate.

If the disorder to be treated is Alzheimer's disease, the other therapeutic agents is preferably a nootropic. The term "nootropic" as used herein includes, but is not limited to calcium antagonists, cholinesterase inhibitors, dihydroergotoxin, nicergoline, piracetame, purine derivates, pyritinol, vincamine and vinpocetine. The term "calcium antagonists" as used herein includes, but is not limited to cinnarizine and nimodipine. The term "cholinesterase inhibitors" as used herein includes, but is not limited to donepezil hydrochloride, rivastigmine and galantamine hydrobromide. The term "purine derivates" as used herein includes, but is not limited to pentifyllin.

If the disorder to be treated is schizophrenia, the other therapeutic agents can be selected from conventional antipsychotics and atypical antipsychotics. The term "conventional antipsychotics" as used herein includes, but is not limited to haloperidol, fluphenazine, thiotixene and flupentixol. The term "atypical antipsychotics" as used herein relates to clozaril, risperidone, olanzapine, quetiapine, ziprasidone and aripiprazol.

If the disorders to be treated are anxiety disorders, the other therapeutic agents can be selected from the group consisting of benzodiazepines, selective serotonin reuptake inhibitors (SSRIs), selective serotonin and norepinephrine reuptake inhibitors (SNRIs), buspirone and pregabalin. The term "benzodiazepines" as used herein includes, but is not limited to clonazepam, diazepam and lorazepam. An SSRI suitable for the present invention is especially selected from fluoxetine, fuvoxamine, sertraline, paroxetine, citalopram and escitalopram. An SNRI suitable for the present invention is especially selected from venlafaxine and duloxetine.

The structure of the active ingredients identified by code nos., generic or trade names may be taken from the actual edition of the standard compendium "The Merck Index" or from databases, e.g. Patents International (e.g. IMS World Publications). The corresponding content thereof is hereby incorporated by reference. Any person skilled in the art is fully enabled to identify the active ingredients and, based on these references, likewise enabled to manufacture and test the pharmaceutical indications and properties in standard test models, both *in vitro* and *in vivo.*

In further aspects, the present invention provides
- a method of treating a disorder or condition in which the mGluR7 receptor plays a role or is implicated, which method comprises administering to a subject in need of such treatment a therapeutically effective amount of a diamine of formula I in free base or pharmaceutically acceptable acid addition salt form;
- a diamine of formula I in free base or pharmaceutically acceptable acid addition salt form, for use as a pharmaceutical; and
- a pharmaceutical composition comprising a diamine of formula I in free base or pharmaceutically acceptable acid addition salt form and at least one pharmaceutically acceptable carrier.

Further, properly isotope-labeled agents of the invention exhibit valuable properties as histopathological labeling agents, imaging agents and/or biomarkers, hereinafter "markers", for the selective labeling of the metabotropic glutamate receptor subtype 7 (mGlu7 receptor). More particularly the agents of the invention are useful as markers for labeling the central and peripheral mGlu7 receptors *in vitro* or *in vivo.* In particular, compounds of the invention which are properly isotopically labeled are useful as PET markers. Such PET markers are labeled with one or more atoms selected from the group consisting of ¹¹C, ¹³N, ¹⁵O, ¹⁸F.

The agents of the invention are therefore useful, for instance, for determining the levels of receptor occupancy of a drug acting at the mGlu7 receptor, or diagnostic purposes for diseases resulting from an imbalance or dysfunction of mGlu7 receptors, and for monitoring the effectiveness of pharmacotherapies of such diseases.

In accordance with the above, the present invention provides an agent of the invention for use as a marker for neuroimaging.

In a further aspect, the present invention provides a composition for labeling brain and peripheral nervous system structures involving mGlu7 receptors *in vivo* and *in vitro* comprising an agent of the invention.

In still a further aspect, the present invention provides a method for labeling brain and peripheral nervous system structures involving mGlu7 receptors *in vitro* or *in vivo,* which comprises contacting brain tissue with an agent of the invention.

The method of the invention may comprise a further step aimed at determining whether the agent of the invention labeled the target structure. Said further step may be effected by observing the target structure using positron emission tomography (PET) or single photon emission computed tomography (SPECT), or any device allowing detection of radioactive radiations.

The following Examples illustrate the invention.

### Abbreviations

Abbreviations used are those conventional in the art and, in particular, have the meanings provided below.
- Ac =: Acetyl
- BAL =: Backbone amide linker
- Bn =: Benzyl
- DCC =: Dicyclohexyl-carbodiimide
- DCE =: 1,2-Dichloroethane
- DCM =: Dichloromethane
- DIC =: Diisopropylcarbodiimide
- DIPEA=: *N*,*N*-Diisopropylethylamine
- DMA =: *N*,*N*-Dimethylacetamide
- DMF =: *N,N*-Dimethylformamide
- DMSO =: Dimethylsulfoxide
- EE =: Ethyl acetate
- ESI-MS=: Electro-spray ionization mass spectroscopy
- HATU=: O-(7-Azobenzotriazol-1-yl)-1,1,3,3-tetramethyluronium hexafluorophosphate
- HOBt =: 1-Hydroxybenzotriazol
- HPLC =: High performance liquid chromatography
- Hx =: Hexanes
- m.p. =: Melting point
- MTBE =: Methyl tert.-butyl ether
- NMR =: Nuclear magnetic resonance spectroscopy
- R_{f} =: Retention factor (TLC)
- RT =: Room temperature
- TFA =: Trifluoroacetic acid
- THF =: Tetrahydrofuran
- TLC =: Thin layer chromatography
- t_{R} =: Retention time (HPLC)

Temperatures are measured in degrees Celsius. Unless indicated otherwise, reactions are carried out at room temperature. The structure of final products, intermediates and starting materials is confirmed by standard analytical methods, e.g. microanalysis and spectroscopic characteristics (e.g. MS, IR, NMR).

### HPLC analysis of final products and intermediates:

**Gradient A:** Performed on a Waters system equipped with a CTC Analytics HTS PAL autosampler, 515 pumps, and a 996 DAD detector operating at 210 nm. Column: CC70/3 Nucleosil 100-3 C₁₈ (3 µ, 70 x 3 mm, Macherey-Nagel, order # 721791.30), temperature: 45°C, flow: 0.8 mL min⁻¹. Eluents: **A**: Water + 0.2% H₃PO₄ (85%, (Merck 100552) + 2% Me₄NOH, (10%, Merck 108123), **B**: Acetonitril + 20% water + 0. 1 % H₃PO₄ (85%) + 1% Me₄NOH (10%). Gradient: 0% B to 95% B within 10 min., then 95% B 5 min. Gradient **B**: Performed on a Waters 2690 system equipped with a 996 DAD detector operating at 220 - 400 nm (Total Plot). Column: CC30/3 Nucleosil 100-3 C₁₈ HD (3 µ, 30 x 3 mm, Macherey-Nagel, order # 721196.30), temperature: 35°C, flow: 1 mL min⁻¹. Eluents: **A**: Water + 0.1% TFA, **B**: Acetonitrile + 0,1% TFA. Gradient: 5% B to 100% B within **4** min., then 100% B 1 min.

**Gradient C**: Performed on a Agilent 1100 series system equipped with DAD detector operating at 254 nm. Column: ZORBAX Eclips XDB C18 (5 µ, 150 x 4.6 mm, Agilent, order # 7995118-595), flow: 1.5 mL min⁻¹. Eluents: **A**: Water + 0.1% TFA, **B**: Acetonitril + 0.1% TFA. Gradient: 0% B to 100% B within 15 min., then 100% B 5 min.

**Gradient D:** Performed on a Agilent 1100 series device and UV detection at 215 nm. Column: Prontosil RP-C₁₈-H (3µ, 53 x 4 mm, Bischoff, order # 0604F185PS030), flow: 1.5 mL min⁻¹. Eluents: **A**: Water + 0.1 % TFA, **B**: Acetonitril + 0.1 % TFA. Gradient: 5% B to 100% B within 10 min.

**Gradient E**: Performed on a Agilent 1100 series device and UV detection at 215 nm. Column: Nucleodur RP-C₁₈-ec (3µ, 53 x 4 mm, Macherey-Nagel, order # 760050.40), flow: 1 mL min⁻¹. Eluents: **A**: Water + 0.1 % TFA, **B**: Acetonitril + 0.1% TFA. Gradient: 5% B to 100% B within 10 min.

### Preparative HPLC separations:

**Gradient F**: Performed on a Waters system equipped with 600 pump, ZQ Micromass MS-detector, and a UV 2487 detector operating at 214 nm. Column: XTerra Prep MS C₁₈ (5 µ 50 x 19 mm, Waters, order # 18600 1930), flow: 20 ml min⁻¹. Eluents: **A**: Water + 0.1% TFA, **B**: Acetonitril + 0.1 % TFA. Gradient: 5% B for 1 min. then 5% B to 95% B within 6 min.

**Gradient G:** Performed on a Shimadzu system equipped with SCL-8A controller, 2x LC-8A pumps, and a Bischoff Lambda 1000 detector operating at 215 nm. Column: Waters Symmetry C18 (5µ 50 x 19 mm, Waters, order # 18600 0210), flow: 20 ml min⁻¹. Eluents: **A**: Water + 0.1 % TFA, **B**: Acetonitril + 0.1 % TFA. Gradient: 5% B to 100% B within 15 min.

### Example 1: 2-(3.4-Dichloro-benzylamino)N,N-diphenyl-acetamide hydrochloride

A solution of chloro-acetylchloride (4.48 ml, 56.3 mmol) in toluene (5 ml) is added at room temperature to a stirred solution of diphenylamine (8.66g 51.1 mmol) and triethylamine (8.6 ml, 61.4 mmol) in toluene (45 ml). The resulting solution is stirred for 90 min. at room temperature and is then brought to reflux for 18 hours. The toluene is evaporated and the residue is dissolved in DCM, washed consecutively with a diluted HCl solution (2N) and brine. The organic layer is dried with sodium sulfate and evaporated. The residue is purified by chromatography (CH₂Cl₂ / hexanes, 4:1) to afford 2-chloro-*N,N*-diphenyl-acetamide as tan crystals (modified method according to R. Sarges et al., J. Med. Chem. 1989 32(2), 437-444).

b) A solution of 2-chloro-*N*,*N*-diphenyl-acetamide (5g, 20.3 mmol), 3,4-dichlorobenzylamine (8.95g, 50.9 mmol) in ethanol (30 ml) is refluxed for 12 hours. The solution is concentrated to about 10 ml and the suspension is filtered off. The filtrate is evaporated and the residue is filtered over silica (CH₂Cl₂ / MeOH, 98:2). The purified fraction is dissolved in a saturated solution of HCl in ethyl acetate from which the hydrochloric salt was filtered off after cooling. The title compound is obtained as colorless crystals: ¹H-NMR (400MHz; DMSO-d₆): δ 3.75 (s, 2H); 4.15 (s , 2H); 7.2-7.6 (m, 11H); 7.7 (d, 1H); 7.8 (d, 1H); 9.55 (NH ,1H), ESI+ MS: m/z = 385.1 (MH⁺).

### Example 2: 2-(2-Chloro-benzylamino)-N,N-diphenyl-acetamide hydrochloride

a) To an ice cooled solution of diphenyl-amine (4.87 g, 28.8 mmol) and pyridine (2.52 ml, 31.4 mmol) in 1,2-dichloroethane (40 ml) is added a solution of bromoacetylbromide (2.5 ml, 30.0 mmol) in 1,2-dichloroethane (10 ml) at such a rate that the internal temperature of the reaction vessel remains below 30 °C. After completion of the addition the ice bath is removed and the reaction mixture is stirred for 1 h at room temperature. Then, the reaction mixture is poured on ice water (100 ml). The mixture is made acidic by addition of 2 M HCl and ethyl acetate (50 ml) is added. The organic layer is separated, washed (2 x water (100ml), 1 x brine (100 ml)), dried over Na₂SO₄, filtered and evaporated *in vacuo.* The obtained brown oil is purified by recrystallization from diethyl ether to provide 2-bromo-N,N-diphenyl-acetamide as off white crystals. (modified method according to F. Oezkanli et al., Arzneim. Forsch. 1994 44(8), 920-924).
b) A solution of 2-bromo-N,N-diphenyl-acetamide (200 mg, 0.69 mmol) and 2-chlorobenzylamine (208 µl, 1.73 mmol) in dry ethanol (1.5 ml) is heated to 80°C with stirring in a 4 ml Pierce Reacti-Vial over night. After reaching room temperature the reaction mixture is poured on a mixture of ethyl acetate (10 ml) and saturated NaHCO₃ solution (10 ml). The organic layer is separated, washed (1× H₂O, 2x brine), dried over Na₂SO₄, and evaporated to dryness. The residue is purified by flash chromatography (FlashMaster chromatography system, 10g Flash-Si cartridge (IST ISOLUTE^{®} SPE, ethyl acetate / hexanes gradient) to give a yellow oil. The free base is dissolved in ethyl acetate (2 ml) and acidified by addition of a few drops of 4M HCI in dioxane. The precipitate is filtered off and washed with diethyl ether. Drying *in vacuo* at 60°C over night give 2-(2-Chloro-benzylamino)-*N,N*-diphenylacetamide hydrochloride as pale yellow powder. M.p. = 213-214.5 °C; HPLC: t_{R} = 6.99 min (gradient A), ESI+ MS: m/z = 351 (MH⁺).

### Examples 3 - 43

Following the same procedures as described under Examples 1 and 2 the following compounds are obtained using suitable starting materials:

### Example 3: 2-(2,2-Diphenyl-ethylamino)-N,N-diphenyl-acetamide hydrochloride

M.p. = 232-234°C; HPLC: t_{R} = 8.18 min (gradient A); ESI+ MS: m/z = 407.5 (MH⁺).

### Example 4: 2-[(Naphthalen-1-ylmethyl)-amino]-N,N-diphenyl-acetamide hydrochloride

M.p. = 233-235°C; HPLC: t_{R} = 7.60 min (gradient A); ESI+ MS: m/z = 367.5 (MH⁺).

### Example 5: N-Benzhydryl-2-(2-chloro-benzylamino)-acetamide

M.p. = 98-99°C; HPLC: t_{R} = 7.56 min (gradient A); ESI+ MS: m/z = 365.3 (MH⁺).

### Example 6: 2-(2-Chloro-benzylamino)-1-(3,4-dihydro-2H-quinolin-1-yl)-ethanone hydro-chloride

M.p. = 182-186°C; HPLC: t_{R} = 6.58 min (gradient A); ESI+ MS: m/z = 315.2 (MH⁺).

### Example 7: 1-(3,4-Dihydro-2H-quinolin-1-yl)-2-(2,2-diphenyl-ethylamino)-ethanone hydrochloride

M.p. = 174-178°C; HPLC: t_{R} = 8.07 min (gradient A); ESI+ MS: m/z = 371.5 (MH⁺).

### Example 8: 2-(2,2-Diphenyl-ethylamino)-N-methyl-N-phenyl-acetamide hydrochloride

M.p. = 221-223°C; HPLC: t_{R} = 6.11 min (gradient A); ESI+ MS: m/z = 345.5 (MH⁺).

### Example 9: 2-(3,4-Dichloro-benzylamino)-N-methyl-N-phenyl-acetamide hydrochloride

M.p. = 221-224 °C; HPLC: t_{R} = 6.04 min (gradient A); ESI+ MS: m/z = 323.2 (MH⁺).

### Example 10: 1-(3,4-Dihydro-1H-isoquinolin-2-yl)-2-(2,2-diphenyl-ethylamino)-ethanone hydrochloride

M.p. = 72-78 °C; HPLC: t_{R} = 7.93 min (gradient A); ESI+ MS: m/z = 371.5 (MH⁺).

### Example 11: 2-(2,2-Diphenyl-ethylamino)-N-isopropyl-N-phenyl-acetamide hydrochloride

M.p. = 240-243 °C; HPLC: t_{R} = 8.19 min (gradient A); ESI+ MS: m/z = 373.5 (MH⁺).

### Example 12: N-Isopropyl-2-[(naphthalen-1-ylmethyl)-amino]-N-phenyl-acetamide hydrochloride

M.p. = 224-226 °C; HPLC: t_{R} = 7.50 min (gradient A); ESI+ MS: m/z = 333.3 (MH⁺).

### Example 13: 2-(3,4-Dichloro-benzylamino)-N-isopropyl-N-phenyl-acetamide hydrochloride

M.p. = 245-250 °C; HPLC: t_{R} = 7.61 min (gradient A); ESI+ MS: m/z = 351.3 (MH⁺).

### Example 14: 2-(3,4-Dihydro-1H-isoquinolin-2-yl)-N,N-diphenyl-acetamide

M.p. = 202-204 °C; HPLC: t_{R} = 7.11 min (gradient A); ESI+ MS: m/z = 343.4 (MH⁺).

### Example 15: 2-(Benzhydryl-amino)-N,N-diphenyl-acetamide

M.p. = 122-123 °C; HPLC: t_{R} = 8.56 min (gradient A); ESI+ MS: m/z = 393.5 (MH⁺).

### Example 16: 2-(2,2-Diphenyl-ethylamino)-1-phenoxazin-10-yl-ethanone hydrochloride

M.p. = 215-216 °C; HPLC: t_{R} = 8.77 min (gradient A); ESI+ MS: m/z = 421.5 (MH⁺).

### Example 17: 1-Carbazol-9-yl-2-(2,2-diphenyl-ethylamino)-ethanone hydrochloride

M.p. = 250 °C; HPLC: t_{R} = 9.06 min (gradient A); ESI+ MS: m/z = 405.3 (MH⁺).

### Example 18: 2-(Benzhydryl-amino)-N-naphthaten-1-yl-acetamide hydrochloride

M.p. = 132-134 °C; HPLC: t_{R} = 8.85 min (gradient A); ESI+ MS: m/z = 367.3 (MH⁺).

### Example 19: 2-(Benzhydryl-amino)-N-biphenyl-2-yl-acetamide hydrochloride

M.p. = 151-158 °C; HPLC: t_{R} = 10.74 min (gradient A); ESI+ MS: m/z = 393.4 (MH⁺).

### Example 20: N-Benzyl-2-(2,2-diphenyl-ethylamino)-N-phenyl-acetamide

### hydrochloride

M.p. = 188-190 °C; HPLC: t_{R} = 8.83 min (gradient A); ESI+ MS: m/z = 421.6 (MH⁺).

### Example 21: N-Benzyl-2-(2-chloro-benzylamino)-N-phenyl-acetamide hydrochloride

M.p. = 190-192 °C; HPLC: t_{R} = 7.63 min (gradient A); ESI+ MS: m/z = 365.4 (MH⁺).

### Example 22: 2-(Benzhydryl-amino)-N-benzyl-N-phenyl-acetamide hydrochloride

M.p. = 181-182 °C; HPLC: t_{R} = 8.97 min (gradient A); ESI+ MS: m/z = 407.6 (MH⁺).

### Example 23: 2-(Benzhydryl-amino)-1-(3,4-dihydro-2H-quinolin-1-yl)-ethanone hydrochloride

M.p. = 191-194 °C; HPLC: t_{R} = 7.90 min (gradient A), ESI+ MS: m/z = 357.6 (MH⁺).

### Example 24: 2-(Benzhydryl-amino)-N-methyl-N-phenyl-acetamide

M.p. = 115-116 °C; HPLC: t_{R} = 7.24 min (gradient A); ESI+ MS: m/z = 331.5 (MH⁺).

### Example 25: 2-(Benzhydryl-amino)-N-isopropyl-N-phenyl-acetamide hydrochloride

M.p. = 241-243 °C; HPLC: t_{R} = 8.09 min (gradient A); ESI+ MS: m/z = 359.6 (MH⁺).

### Example 26: 2-(2,2-Diphenyl-ethylamino)-1-(8-methyl-3,4-dihydro-2H-quinolin-1-yl)-ethanone hydrochloride

M.p. = 82-95°C; HPLC: t_{R} = 8.25 min (gradient A); ESI+ MS: m/z = 385.3 (MH⁺).

### Example 27: 2-(Benzhydryl-amino)-1-(8-methyl-3,4-dihydro-2H-quinolin-1-yl)-

### ethanone hydrochloride

M.p. = 128-136°C; HPLC: t_{R} = 8.22 min (gradient A), ESI+ MS: m/z = 371.3 (MH⁺).

### Example 28: rac-2-(2,2-Diphenyl-ethylamino)-1-(2-methyl-3,4-dihydro-2H-quinolin-1 yl)-ethanone hydrochloride

M.p. = 212-215°C; HPLC: t_{R} = 8.21 min (gradient A), ESI+ MS: m/z = 385.5 (MH⁺).

### Example 29: 1-(3,4-Dihydro-2H-quinolin-1-yl)-2-(1,1-dimethyl-2-phenyl-ethylamino)-ethanone hydrochloride

Yellow gum, HPLC: t_{R} = 7.23 min (gradient A), ESI+ MS: m/z = 323.4 (MH⁺).

### Example 30: 1-(3,4-Dihydro-2H-quinolin-1-yl)-2-(1-methyl-1-phenyl-ethylamino)-ethanone hydrochloride

Yellow gum, HPLC: t_{R} = 6.65 min (gradient A), ESI+ MS: m/z = 309.5 (MH⁺).

### Example 31: 1-(3,4-Dihydro-2H-quinolin-1-yl)-2-(9H-fluoren-9-ylamino)-ethanone

M.p. = 82-84°C; HPLC: t_{R} = 7.75 min (gradient A), ESI+ MS: m/z = 355.6 (MH⁺).

### Example 32: 1-(3,4-Dihydro-2H-quinolin-1-yl)-2-(2,2-diphenyl-propylamino) ethanone hydrochloride

M.p. = 210-212°C; HPLC: t_{R} = 8.20 min (gradient A), ESI+ MS: m/z = 385.8 (MH⁺).

### Example 33: 2-(3,4-Dihydro-1H-isoquinolin-2-yl)-1-(3,4-dihydro-2H-quinolin-1-yl)-ethanone hydrochloride

M.p. = 201-203°C; HPLC: t_{R} = 6.63 min (gradient A), ESI+ MS: m/z = 307.3 (MH⁺).

### Example 34: 1,3-Bis-(3,4-dihydro-2H-quinolin-1-yl)-propan-1-one

Pale brown syrup, HPLC: t_{R} = 10.57 min (gradient A), ESI+ MS: m/z = 321.4 (MH⁺).

### Example 35: rac-N,N-Diphenyl-2-(1-phenyl-ethylamino)-acetamide hydrochloride

M.p. = 232-236°C; ¹H-NMR (400MHz; CDCl₃): δ 1.36 (d, J=8 Hz, 3H), 3.13 (s, 2H), 3.77 (q, J=8 Hz, 1 H), 7.08 - 7.35 (m, 15H); ESI+ MS: m/z = 331.2 (MH⁺).

### Example 36: 1-Dibenzo[b,f]azepin-5-yl-2-(1,2,3,4-tetrahydro-naphthalen-1-ylamino)-ethanone hydrochloride

M.p. = 245-246°C; HPLC: t_{R} = 2.65 min (gradient B), ESI+ MS: m/z = 381.2 (MH⁺).

### Example 37: 2-Benzylamino-N,N-diphenyl-acetamide hydrochloride

M.p. = 234-237°C; HPLC: t_{R} = 2.40 min (gradient B), ESI+ MS: m/z = 317.2 (MH⁺).

### Example 38: 1-(10,11-Dihydro-dibenzo[b,f]azepin-5-yl)-2-phenethylamino-ethanone hydrochloride

HPLC: t_{R} = 2.64 min (gradient B), ESI+ MS: m/z = 357.2 (MH⁺).

### Example 39: 1-Dibenzo[b,f]azepin-5-yl-2-(8-methoxy-1,2,3,4-tetrahydro-naphthalen 2-ylamino)-ethanone hydrochloride

M.p. = 167-271°C; HPLC: t_{R} = 2.73 min (gradient B), ESI+ MS: m/z = 411.2 (MH⁺).

### Example 40: 2-(3,3-Diphenyl-propylamino)-N,N-diphenyl-acetamide hydrochloride

M.p. = 227-230°C; HPLC: t_{R} = 2.94 min (gradient B), ESI+ MS: m/z = 421.2 (MH⁺).

### Example 41: 2-Phenethylamino-N,N-diphenyl-acetamide hydrochloride

M.p. = 250-253°C; HPLC: t_{R} = 2.48 min (gradient B), ESI+ MS: m/z = 331.2 (MH⁺).

### Example 42: 1-Dibenzo[b,f]azepin-5-yl-2-(3,4-dichloro-benzylamino)-ethanone hydrochloride

M.p. = 244-249°C; HPLC: t_{R} = 2.82 min (gradient B), ESI+ MS: m/z = 409.0 (MH⁺).

### Example 43: 1-Dibenzo[b,f]azepin-5-yl-2-phenethylamino-ethanone hydrochloride

M.p. = 243-259°C; HPLC: t_{R} = 2.58 min (gradient B), ESI+ MS: m/z = 355.2 (MH⁺).

### Example 44: rac-1-(3,4-Dihydro-2H-quinolin-1-yl)-2-(1-phenyl-ethylamino)-ethanone trifluoro acetate

a) 2-Bromo-1-(3,4-dihydro-2*H*-quinolin-1-yl)-ethanone is prepared analogously to bromo-N,N-diphenyl-acetamide (Example 1, step a) from 1,2,3,4-tetrahydroquinoline (7.89 ml, 59.1 mmol), pyridine (5.23 ml, 65.0 mmol) and bromoacetylbromide (5.77 ml, 65 mmol) in 1,2-dichloroethane (60 ml). The title compound is obtained as greenish oil.
b) To a solution of 2-bromo-1-(3,4-dihydro-2H-quinolin-1-yl)-ethanone (25 mg, 0.1 mmol) and *rac*-1-phenylethylamine (25 µl, 0.2 mmol) in N,N-dimethyl acetamide (0.6 ml) is added a 30% Na₂CO₃ solution in water (75 µl, 0.21 mmol). The reaction mixture is shaken in a 4.5 ml fritted polypropylene reaction tube for 24h at 50 °C (Mettler-Toledo Bohdan 48 position MiniBlock^{™} synthesizer). Then, the solution is filtered and directly submitted to preparative HPLC (gradient F). The title compound is obtained as a yellow gum; HPLC: t_{R} = 6.64 min (gradient A); ESI+ MS: m/z = 295 (MH⁺).

### Examples 45 - 82

Following the same procedures as described under Example 44 the following compounds are obtained using suitable starting materials:

### Example 45: 1-(3,4-Dihydro-2H-quinolin-1-yl)-2-(1-naphthalen-1-yl-ethylamino) ethanone trifluoro acetate

HPLC: t_{R} = 7.40 min (gradient A), ESI+ MS: m/z = 345.5 (MH⁺).

### Example 46: 1-(3,4-Dihydro-2H-quinolin-1-yl)-2-(2-trifluoromethoxy-benzylamino)-ethanone trifluoro acetate

HPLC: t_{R} = 7.24 min (gradient A); ESI+ MS: m/z = 365.3 (MH⁺).

### Example 47: (R)-1-(3,4-Dihydro-2H-quinolin-1-yl)-2-[methyl-(1-phenyl-ethyl)-amino]-ethanone trifluoro acetate

HPLC: t_{R} = 6.59 min (gradient A), ESI+ MS: m/z = 309.4 (MH⁺).

### Example 48: 2-[1-(4-Chloro-phenyl)-ethylamino]-1-(3,4-dihydro-2H-quinolin-1-yl)-ethanone trifluoro acetate

HPLC: t_{R} = 7.06 min (gradient A), ESI+ MS: m/z = 329.3 (MH⁺).

### Example 49: 2-(Chroman-4-ylamino)-1-(3,4-dihydro-2H-quinolin-1-yl)-ethanone trifluoro acetate

HPLC: t_{R} = 6.54 min (gradient A), ESI+ MS: m/z = 323.4 (MH⁺).

### Example 50: N,N-Diphenyl-2-(2-trifluoromethoxy-benzylamino)-acetamide trifluoro acetate

HPLC: t_{R} = 7.69 min (gradient A), ESI+ MS: m/z = 401.6 (MH⁺).

### Example 51: 2-[2-(1H-Indol-3-yl)-ethylamino]-N,N-diphenyl-acetamide trifluoro acetate

HPLC: t_{R} = 7.34 min (gradient A), ESI+ MS: m/z = 370.6 (MH⁺).

### Example 52: 2-(2-Hydroxy-2-phenyl-ethylamino)-N,N-diphenyl-acetamide trifluoro acetate

HPLC: t_{R} = 6.65 min (gradient A), ESI+ MS: m/z = 347.4 (MH⁺).

### Example 53: 2-[1-(4-Chloro-phenyl)-ethylamino]-N,N-diphenyl-acetamide trifluoro acetate

HPLC: t_{R} = 7.54 min (gradient A), ESI+ MS: m/z = 365.4 (MH⁺).

### Example 54: rel-(1R,2aS)-2-(1,2,2a,3,4,5-Hexahydro-acenaphthylen-1-ylamino)-N,N-diphenyl-acetamide trifluoro acetate

HPLC: t_{R} = 7.70 min (gradient A), ESI+ MS: m/z = 383.6 (MH⁺).

### Example 55: 2-(2,3-Dimethyl-benzylaminol-N,N-diphenyl-acetamide trifluoro acetate

HPLC: t_{R} = 7.50 min (gradient A), ESI+ MS: m/z = 345.5 (MH⁺).

### Example 56: 2-(2,4-Dichloro-benzylamino)-N,N-diphenyl-acetamidetrifluoro acetate

HPLC: t_{R} = 7.48 min (gradient A), ESI+ MS: m/z = 385.5 (MH⁺).

### Example 57: trans-2-(1-Methyl-3-phenyl-piperldin-4-ylamino)-N,N-diphenylacetamide trifluoro acetate

HPLC: t_{R} = 7.74 min (gradient A), ESI+ MS: m/z = 400.7 (MH⁺).

### Example 58: trans-N,N-Diphenyl-2-(2-phenyl-cyclopropylamino)-acetamide trifluoro acetate

HPLC: t_{R} = 7.46 min (gradient A), ESI+ MS: m/z = 343.4 (MH⁺).

### Example 59: (S)-2-(8-Methoxy-1,2,3,4-tetrahydro-naphthalen-2-ylamino)-N,N-diphenyl-acetamide trifluoro acetate

HPLC: t_{R} = 7.45 min (gradient A), ESI+ MS: m/z = 387.7 (MH⁺).

### Example 60: 2-(1,2-Diphenyl-ethylamino)-N,N-diphenyl-acetamide trifluoro acetate

HPLC: t_{R} = 8.33 min (gradient A), ESI+ MS: m/z = 407.7 (MH⁺).

### Example 61: rac-2-(1-Naphthalen-1-yl-ethylamino)-N,N-diphenyl-acetamide trifluoro acetate

HPLC: t_{R} = 7.82 min (gradient A), ESI+ MS: m/z = 381.7 (MH⁺).

### Example 62: 2-(2-Chloro-6-methyl-benzylamino)-N,N-diphenyl-acetamidetrifluoro acetate

HPLC: t_{R} = 7.48 min (gradient A), ESI+ MS: m/z = 365.5 (MH⁺).

### Example 63: rac-2-(Acenaphthen-1-ylamino)-1-(3,4-dihydro-2H-quinolin-1-yl)-ethanone trifluoro acetate

HPLC: t_{R} = 7.38 min (gradient A), ESI+ MS: m/z = 343.4 (MH⁺).

### Example 64: 2-(2-Bromo-benzylamino)-N,N-diphenyl-acetamide trifluoro acetate

HPLC: t_{R} = 7.37 min (gradient A), ESI+ MS: m/z = 395.3 (MH⁺).

### Example 65: 2-(2-Methyl-benzylamino)-N,N-diphenyl-acetamide trifluoro acetate

HPLC: t_{R} = 7.33 min (gradient A), ESI+ MS: m/z = 331.4 (MH⁺).

### Example 66: N,N-Diphenyl-2-(2-trifluoromethyl-benzylamino)-acetamide trifluoro acetate

HPLC: t_{R} = 7.69 min (gradient A), ESI+ MS: m/z = 385.4 (MH⁺).

### Example 67: 2-(2-Ethoxy-benzylamino)-N,N-diphenyl-acetamide trifluoro acetate

HPLC: t_{R} = 7.61 min (gradient A), ESI+ MS: m/z = 361.4 (MH⁺).

### Example 68: 2-[(Biphenyl-2-ylmethyl)-aminol-N,N-diphenyl-acetamide trifluoro acetate

HPLC: t_{R} = 8.21 min (gradient A), ESI+ MS: m/z = 393.5 (MH⁺).

### Example 69: rac-2-(Acenaphthen-1-ylamino)-N,N-diphenyl-acetamide trifluoro acetate

HPLC: t_{R} = 7.90 min (gradient A), ESI+ MS: m/z = 379.4 (MH⁺).

### Example 70: (S)-1-(3,4-Dihydro-2H-quinolin-1-yl)-2-(1-Phenyl-ethylamino)-ethanone trifluoro acetate

HPLC: t_{R} = 6.51 min (gradient A), ESI+ MS: m/z = 295.4 (MH⁺).

### Example 71: (R)-1-(3,4-Dihydro-2H-quinolin-1-yl)-2-(1-phenyl-ethylamino)-ethanone trifluoro acetate

HPLC: t_{R} = 6.54 min (gradient A), ESI+ MS: m/z = 295.4 (MH⁺).

### Example 72: rac-5,6,11,12-Tetrahydro-2-methoxy-14-(2,2-diphenylethyl)amino)acetyl-dibenzo[a,e]cycloocten-5,11-imine trifluoro acetate

HPLC: t_{R} = 8.83 min (gradient A), ESI+ MS: m/z = 489.7 (MH⁺).

### Example 73: (4'S,5S,10R)-12-(Chroman-4-ylamino)acetyl-10,11-dihydro-5-methyl-5H-dibenzo[a,d]cyclohepten-5-10-imine trifluoro acetate

HPLC: t_{R} = 7.93 min (gradient A), ESI+ MS: m/z = 411.6 (MH⁺).

### Example 74: 2-(Benzhydryl-amino)-1-(6-mothyl-3,4-dihydro-2H-quinolin-1-yl)-ethanone trifluoro acetate

HPLC: t_{R} = 5.23 min (gradient A), ESI+ MS: m/z = 371.2 (MH⁺).

### Example 75: 2-(2,2-Diphenyl-ethylamino)-1-(6-methyl-3,4-dihydro-2H-quinolin-1-yl)-ethanone trifluoro acetate

HPLC: t_{R} = 5.51 min (gradient A), ESI+ MS: m/z = 385.6 (MH⁺).

### Example 76: 2-(3,4-Dichloro-benzylamino)-1-(6-methyl-3,4-dihydro-2H-quinolin-1-yl)-ethanonetrifluoro acetate

HPLC: t_{R} = 5.11 min (gradient A), ESI+ MS: m/z = 364.3 (MH⁺).

### Example 77: rac-1-(6-Methyl-3,4-dihydro-2H-quinolin-1-yl)-2-(1-naphthaten-1-yl-ethyl-amino)-ethanone trifluoro acetate

HPLC: t_{R} = 5.16 min (gradient A), ESI+ MS: m/z = 359.1 (MH⁺).

### Example 78: (S)-2-(Chroman-4-ylamino)-1-(6-methyl-3,4-dihydro-2H-quinolin-1-yl)-ethanone trifluoro acetate

HPLC: t_{R} = 4.63 min (gradient A), ESI+ MS: m/z = 337.0 (MH⁺).

### Example 79: (S)-2-(Chroman-4-ylamino)-1-(7-trifluoromethyl-3,4-dihydro-2H-quinolin-1-yl)-ethanone trifluoro acetate

HPLC: t_{R} = 4.97 min (gradient A), ESI+ MS: m/z = 391.0 (MH⁺).

### Example 80: rac-2-(Benzhydryl-amino)-1-(6-fluoro-2-methyl-3,4-dihydro-2H-quinolin-1-yl)-ethanone trifluoro acetate

HPLC: t_{R} = 5.26 min (gradient A), ESI+ MS: m/z = 389.1 (MH⁺).

### Example 81: rac-2-(Benzhydryl-amino)-1-(2,2,4,7-tetramethyl-3,4-dihydro-2H-quinolin-1-yl)-ethanone trifluoro acetate

HPLC: t_{R} = 6.02 min (gradient A), ESI+ MS: m/z = 413.1 (MH⁺).

### Example 82: (R)-1-(3,4-Dihydro-2H-quinolin-1-yl)-2-(1-phenyl-propylamino)-ethanone trifluoro acetate

HPLC: t_{R} = 4.51 min (gradient A), ESI+ MS: m/z = 309.0 (MH⁺).

### Example 83: 2-(Benzhydryl-amino)-N-(3,3-diphenyl-propyl)-acetamide hydrochloride

a) To a solution of α-aminodiphenylmethane (3.40 ml, 20 mmol) in chloroform (20 ml) is added triethylamine (1.4 ml, 10 mmol), ethyl bromoacetate (1.1 ml, 10 mmol), and molecular sieves 4A. The reaction mixture is shaken at room temperature under argon for 20h (Büchi Syncore apparatus). Then, the molecular sieves is filtered off and the filtrate extracted once with water. The aqueous layer is washed with chloroform once. The combined organic layers are dried (Na₂SO₄) and evaporated to give a yellow syrup. The crude product is purified by flash chromatography to afford ethyl (benzhydryl-amino)-acetate as a colorless oil.
b) A solution of ethyl (benzhydryl-amino)-acetate (781 mg, 2.9 mmol) in 2M KOH in methanol (5 ml, 10 mmol) is refluxed for 2h. Then, the solvent is evaporated and the residue re-dissolved in water. The pH of the solution is adjusted to 7.3 by addition of 2M HCl. The precipitated crystals are collected, washed with water and high vacuum dried to give (benzhydryl-amino)-acetic acid as colorless crystals.
c) To a solution of (benzhydryl-amino)-acetic acid (139 mg, 0.5 mmol) in DMF (5 ml) is added triethylamine (209 µl, 1.5 mmol), HOBt (122 mg, 0.9 mmol), DIC (116 µl, 0.75 mmol), and 3,3-diphenylpropylamine (106 mg, 0.5 mmol). The reaction mixture is shaken on a Büchi Syncore reactor for 72h at room temperature. The mixture is diluted with water and extracted twice with toluene. The combined organic layers are dried (Na₂SO₄) and evaporated under reduced pressure. The residue is dissolved in ether and filtered over Hyflo. To the clear filtrate 4 M HCl in dioxane (125 µl, 0.5 mmol) is added and the suspension obtained is stirred for 15 min. The white precipitate is filtered off, washed with ether and high vacuum dried to afford the title compound as colorless crystals: M.p. = 177.5-178.1 °C; HPLC: t_{R} = 11.52 min (gradient C), ESI+ MS: m/z = 435.2 (MH⁺).

### Example 84: 2-(Benzhydryl-amino)-N-benzyl-acetamide hydrochloride

The title compound is prepared by coupling of (benzhydryl-amino)-acetic acid (example 44, step b) with benzylamine according to the procedure given in example 44, step c) and is obtained as colorless crystals: M.p. = 161.9-162.9 °C; HPLC: t_{R} = 12.08 min (gradient C), ESI+ MS: m/z = 330.9 (MH⁺).

### Example 85: 2-[2-(2-Hydroxy-ethoxy)-benzylamino]-N,N-diphenyl-acetamide hydrochloride

a) 2-Amino-N,N-diphenyl-acetamide: A solution of 2-chloro-N,N-diphenyl-acetamide (8.5 g, 34.6 mmol, Example 1, step a) in methanol saturated with ammonia (800 ml, 2.8 M) is stirred at 60 °C for 19 hours. Then another 80 ml of the ammonia in methanol is added and stirring is continued for 24 hours at.60 °C. Evaporation of the solvent and chromatography on silica gel (DCM / methanol gradient) yields pure fractions of a yellow oil which, upon standing solidifies. M.p. = 108-110°C.
b) 2-[2-(2-Hydroxy-ethoxy)-benzylamino]-N,N-diphenyl-acetamide hydrochloride: To a solution of 2-amino-N,N-diphenyl-acetamide (102 mg, 0.451 mmol) in methanol (1 ml) is added 2-(2-hydroxy-ethoxy)-benzaldehyde (50 mg, 0.301 mmol), acetic acid (26 µl, 0.451 mmol) and sodium triacetoxborohydride (191 mg, 0.903 mmol). After stirring the mixture for 1.5 hours at room temperature the solvent is removed in vacuo and the residue is dissolved in DCM, washed consecutively twice with an aqueous sodium hydroxide solution (1M) and brine. The organic layer is dried with sodium sulfate and evaporated. The residue is purified by chromatography on silica gel (DCM / methanol gradient) and the obtained title compound converted into its hydrochloride salt by treatment of a dioxan solution with hydrogen chloride in dioxan. M.p. = 90-160°C; HPLC: t_{R} = 2.34 min (gradient B), ESI+ MS: m/z = 377.2 (MH⁺).

### Example 86: N,N-Diphenyl-2-(1,2,3,4-tetrahydro-naphthalen-2-ylamino)-acetamide hydrochloride

The title compound is obtained according to the procedure described in Example 85.; m.p. = 274-295°C; HPLC: t_{R} = 2.60 min (gradient B), ESI+ MS: m/z = 357.2 (MH⁺).

### Example 87: 2-Amino-N-[(diphenylcarbamoyl)-methyl]-2-phenyl-acetamide hydrochloride

a) (R)-({[(Diphenylcarbamoyl)-methyl]-carbamoyl}-phenyl-methyl)-carbamic acid tert-butyl ester: To a suspension of (R)- *tert*-butoxycarbonylamino-phenyl-acetic acid (100 mg, 0.398 mmol) and 2-amino-N,N-diphenyl-acetamide (90 mg, 0.398 mmol, Example 85, step a) in DCM/THF (1.5 ml, 2:1) is added pyridine (6 µl, 0.08 mmol), DCC (90 mg, 0.438 mmol) and HOBT (54mg, 0.398 mmol) and the mixture is stirred at room temperature for 16 hours. The reaction suspension is filtered, the filtrate diluted with ethyl acetate and washed consecutively three times with a saturated aqueous solution of sodium hydrogen carbonate. The organic layer is dried with sodium sulfate and evaporated to yield the desired product as a yellow oil which is used in the next step without further purification. HPLC: t_{R} = 3.14 min (gradient B), ESI+ MS: m/z = 482.2 (M•Na⁺).
b) (R)-2-Amino-*N*-[(diphenylcarbamoyl)-methyl]-2-phenyl-acetamide: ({[(Diphenylcarbamoyl)-methyl]-carbamoyl}-phenyl-methyl)-carbamic acid tert-butyl ester (95 mg, 0.207 mol) is treated with 1 ml of a solution of hydrogen chloride in dioxan (4 M) for 10 minutes. The light brown oil obtained after evaporation of the solvent is crystallized from DCM and diethyl ether to afford the title compound as a white powder. M.p. = 177-185°C (dec.); ¹H-NMR (400MHz; CDCl₃): δ 3.94 (d, J=7 Hz, 2H), 4.53 (s, 1 H), 7.10 - 7.45 (m, 15H), 7.82 (b, 1H); ESI+ MS: m/z = 360.2 (MH⁺).

### Example 88: (R)-2-Amino-N-(2-dibenzo[b,f]azepin-5-yl-2-oxo-ethyl)-2-phenyl-acetamide hydrochloride

a) 2-Amino-1-dibenzo[b,f]azepin-5-yl-ethanone: This compound is prepared in a similar way as in Example 85, step a). HPLC: t_{R} = 2.07 (gradient B), ESI+ m/z = 251.2 (MH⁺).
b) (R)-[(2-Dibenzo[b,f]azepin-5-yl-2-oxo-ethylcarbamoyl)-phenyl-methyl]-carbamic acid tert-butyl ester: This compound is prepared in a similar way as in Example 87 step a). HPLC: t_{R} = 3.14 (gradient c), ESI+ m/z = 506.2 (M•Na⁺).
c) (R)-2-Amino-N-(2-dibenzo[b,f]azepin-5-yl-2-oxo-ethyl)-2-phenyl-acetamide hydrochloride: This compound is prepared in a similar way as in example 87 step b). M.p. 179 - 185 °C; HPLC: t_{R} = 2.43 (gradient B), ESI+ m/z = 384.0 (MH⁺).

### Example 89: (S)-2-Ammo-N-(2-dibenzo[b,f]azepin-5-yl-2-oxo-ethyl)-2-phenyl-acetamide hydrochloride

This compound is prepared in accordance with the procedures as described in Example 88. M.p. 192-233 °C; HPLC: t_{R} = 2.43 (gradient B), ESI+ m/z = 384.2 (MH⁺).

### Examples 90 - 91

The following examples are prepared according to the procedure outlined in Example 2.

### Example 90: rac-2-(2,2-Diphenyl-ethylamino)-N,N-diphenyl-propionamide hydrochloride

M.p. = 122-125°C; HPLC: t_{R} = 8.79 min (gradient A), ESI+ MS: m/z = 421.6 (MH⁺).

### Example 91: rac-2-(Indan-2-ylamino)-N,N-diphenyl-propionamide hydrochloride

M.p. = 208-210°C; HPLC: t_{R} = 7.61 min (gradient A), ESI+ MS: m/z = 357.6 (MH⁺).

### Examples 92 - 94

The following examples are prepared according to the procedure outlined in Example 44.

### Example 92: rac-2-(2-Methyl-benzylamino)-N,N-diphenyl-propionamide trifluoro acetate

HPLC: t_{R} = 7.47 min (gradient A), ESI+ MS: m/z = 345.5 (MH⁺).

### Example 93: rac-N,N-Diphenyl-2-(2-trifluoromethyl-benzylamino)-propionamide trifluoro acetate

HPLC: t_{R} = 8.08 min (gradient A), ESI+ MS: m/z = 399.4 (MH⁺).

### Example 94: rac-2-[(Biphenyl-2-ylmethyl)-amino]-N,N-diphenyl-propionamide trifluoro acetate

HPLC: t_{R} = 8.40 min (gradient A), ESI+ MS: m/z = 407.6 (MH⁺).

### Examples 95 rac-2-(Benzhydryl-amino)-N-(3,3-diphenyl-propyl)-propionamide hydrochloride

The title compound is prepared according to the procedure outlined in Example 83. Colorless crystals: m.p. = 210.3-212.0 °C; HPLC: t_{R} = 15.92 min (gradient C), ESI+ MS: m/z = 448.9 (MH⁺).

### Examples 96 - 98

The following examples are prepared according to the procedure outlined in Example 44.

### Example 96: rac-3-[(Naphthalen-1-ylmethyl)-amino]-N,N-diphenyl-propionamide hydrochloride

M.p. = 192-198°C; HPLC: t_{R} = 7.91 min (gradient A), ESI+ MS: m/z = 382.1 (MH⁺).

### Example 97: rac-3-(Indan-2-ylamino)-N,N-diphenyl-propionamide hydrochloride

M.p. = 239-241°C; HPLC: t_{R} = 7.50 min (gradient A), ESI+ MS: m/z = 358.2 (MH⁺).

### Example 98: rac-3-(2,2-Diphenyl-ethylamino)-N,N-diphenyl-propionamide hydrochloride

M.p. = 198-200°C; HPLC: t_{R} = 8.57 min (gradient A), ESI+ MS: m/z = 422.5 (MH⁺).

### Example 99: rac- N-Benzyl-2-benzylamino-2-phenyl-acetamide fumarate

a) *tert*.-Butyl benzylcarbamoyl-phenyl-methyl)-carbamate: A solution of *tert*.-butoxycarbonyl-amino-phenyl-acetic acid (7.54 g, 30 mmol), 4-*N,N*-dimethylamino-pyridine (1.83 g, 15mmol) in THF (150 ml) is stirred for 5 minutes at room temperature. Di-(N-succinimidyl)-carbonate (7.69 g, 30 mmol) is added and the resulting mixture is stirred for 18 hours. Benzylamine (3.2 g, 30 mmol) is added and the solution is stirred for another 18 hours at room temperature. The resulting suspension is filtered and the filtrate is evaporated. The residue is dissolved in dichloromethane and washed successively with a NaHSO₄ solution (2N), a saturated K₂CO₃ solution and brine. The organic layer is dried over MgSO₄ and evaporated. The crude product is recrystallized from ether to give the desired compound.
b) 2-Amino-N-benzyl-2-phenyl-acetamide: (Benzylcarbamoyl-phenyl-methyl)-carbamic acid tert-butyl ester (8.7 g, 25.4 mmol) is dissolved in HCI saturated ethyl-acetate (100 ml). The solution is stirred at room temperature over night. The solution is concentrated and the resulting crystals are dissolved in CH₂CI₂ and washed with a saturated solution of K₂CO₃-The organic layer is dried and evaporated to afford the desired compound.
c) *N*-Benzyl-2-benzylamino-2-phenyl-acetamide fumarate: 2-Amino-N-benzyl-2-phenyl-acetamide (529 mg, 2.2 mmol), benzaldehyde (202 µl, 2 mmol) are stirred for 2 hours at room temperature in a solution of 1,2-dichloroethane, methanol and acetic acid (6:3:1, 20 ml). Polymer supported cyano-borohydride (1g, 4.3 mmol) is added and the mixture is stirred for 18 hours. The suspension is filtered, the filtrate is evaporated and the residue is purified by chromatography (CH₂Cl₂ / MeOH, 99:1) to afford the desired compound. The compound is dissolved in ethanol and fumaric acid (1 mole-equivalent) is added. The solution is brought to its boiling point and cooled to afford the fumarate salt. ESI+ MS: m/z = 331.2 (MH⁺).

### Examples 100 - 113

The following compounds are prepared in accordance to the procedures as described in Example 99 starting with the appropriate amines and aldehydes:

### Example 100: rac-N-(4-Chloro-benzyl)-2-(4-dimethylamino-benzylamino)-2-phenyl-acet-amide

ESI+ MS: m/z = 408.2 (MH⁺).

### Example 101: rac-N-(4-Chloro-benzyl)-2-(4-nitro-benzylamino)-2-phenyl-acetamide

ESI+ MS: m/z = 410.1 (MH⁺).

### Example 102: rac-N-(2,2-Diphenyl-ethyl)-2-phenyl-2-[(pyridin-2-ylmethyl)-amino]-acetamide

Colorless crystals, ESI+ MS: m/z = 422.2 (MH⁺).

### Example 103: rac-N-Benzyl-2-(4-methoxy-benzylamino)-2-phenyl-acetamide fumarate

Colorless crystals, mp. 153-155°C, ESI+ MS: m/z = 361.3 (MH⁺).

### Example 104: rac-N-(4-Chloro-benzyl)-2-phenyl-2-(4-trifluoromethyl-benzylamino)-acetamide fumarate

Colorless crystals, ESI+ MS: m/z = 433.2 (MH⁺).

### Example 105: rac-N-(4-Chloro-benzyl)-2-(4-methoxy-benzylamino)-2-phenyl-acetamide fumarate

Colorless crystals, ESI+ MS: m/z = 395.2 (MH⁺).

### Example 106: rac-N-Benzyl-2-dibenzylamino-2-phenyl-acetamide

Colorless crystals, mp. 109-119.5°C, ESI+ MS: m/z = 421.2 (MH⁺).

### Example 107: rac-N-Benzyl-2-(4-dimethylamino-benzylamino)-2-phenyl-acetamide fumarate

Yellow crystals, mp. 159-161 °C, ESI+ MS: m/z = 374.3 (MH⁺).

### Example 108: rac-N-Benzyl-2-(4-chloro-benzylamino)-2-phenyl-acetamide fumarate

Colorless crystals, mp. 142-205°C (dec.), ESI+ MS: m/z = 365.2 (MH⁺).

### Example 109: rac-N-(4-Chloro-benzyl)-2-(2,4-dichloro-benzylamino)-2-phenyl-acetamide fumarate

Colorless crystals, ESI+ MS: m/z = 433.1 (MH⁺).

### Example 110: rac-2-Amino-N-(2,2-diphenyl-ethyl)-2-phenyl-acetamide

Colorless crystals, ESI+ MS: m/z = 331.2 (MH⁺).

### Example 111: rac-N-(4-Chloro-benzyl)-2-[4-(3-dimethylamino-propoxy)-benzylaminol-2-phenyl-acetamide fumarate

Colorless crystals, ESI+ MS: m/z = 466.2 (MH⁺)

### Example 112: rac-N-Benzyl-2-(3,5-dimethoxy-benzylamino)-2-phenyl-acetamide

Colorless crystals, mp. 107-108°C, ESI+ MS: m/z = 391.3 (MH⁺)

### Example 113: rac-N-Benzyl-2-(4-chloro-benzylamino)-N-methyl-2-phenyl-acetamide

The title compound is prepared in the same manner starting from 2-Amino-N-benzyl-N-methyl-2-phenyl-acetamide: Colorless crystals, ESI+ MS: m/z = 379.2 (MH⁺)

### Example 114: rac-N-Benzyl-2-[(4-chloro-benzyl)-methyl-amino]-2-phenyl-acetamide fumarate

N-Benzyl-2-(4-chloro-benzylamino)-2-phenyl-acetamide (730 mg, 2 mmol, example 8u), formaldehyde (1 ml, 37% aq. solution) are stirred for 2 hours at room temperature in a solution of dichloroethylene, methanol and acetic acid (6:3:1, 20 ml). Polymer supported cyano-borohydride (1g, 4.3 mmol) is added and the mixture is stirred for 18 hours. The suspension is filtered, the filtrate is evaporated and the residue is purified by chromatography (CH₂Cl₂ / MeOH, 99:1) to afford the desired compound as colorless crystals; 1H-NMR (400MHz; DMSO-d6): 2.05 (s, 3H); 3.4 (dd, 2H); 4.08 (s , 1H); 4.3 (dd, 2H); 7.1-7.4 (m, 12H); 7.48 (s, 1H); 7.51 (s, 1H); ESI+ MS: m/z = 379.2 (MH⁺).

### Example 115: rac-2-[Acetyl-(4-chloro-benzyl)-aminol-N-benzyl-2-phenyl-acetamide

N-Benzyl-2-(4-chloro-benzylamino)-2-phenyl-acetamide (730 mg, 2 mmol, Example 108) and triethylamine (348 µl, 2.5 mmol) is dissolved in methylenechloride (20 ml) and cooled to - 20 °C. Acetylbromide (163 µl, 2.2 mmol) in methylenechloride (1 ml) is added slowly and the resulting solution is stirred for 30 min. and is then warmed to 0 °C and stirred for another 90 min. The solution is warmed to room temperature and is stirred overnight. The mixture is - washed with a diluted HCl solution (2N), dried and evaporated. The residue is purified by chromatography (CH₂CI₂ / MeOH, 99:1) and the enriched fraction is recrystallized from ether to afford the desired compound. ESI+ MS: m/z = 407.2 (MH+)

### Examples 116 - 118

The following compounds are prepared in accordance to the procedures as described in Example 115 starting with the appropriate starting materials:

### Example 116: rac-N-(Benzylcarbamoyl-phenyl-methyl)-4-cyano-benzamide

Colorless crystals, mp. 176-178°C, ESI+ MS: m/z = 368.2 (MH⁺).

### Example 117: 6-[(Benzylcarbamoyl-phenyl-methyl)-carbamoyl]-1H-indole-2-carboxylic acid; mp. 258-260°C.

### Example 118: {6-[(Benzylcarbamoyl-phenyl-methyl)-carbamoyl]-benzofuran-3-yl}-acetic acid methyl ester

Prepared from 2-Amino-*N*-benzyl-2-phenyl-acetamide (example 8a) and 3-Methoxycarbonylmethyl-benzofuran-6-carboxylic acid; mp. 129-131°C.

### Examples 119 - 120

The following examples are prepared according to the procedure outlined in Example 44:

### Example 119: rac-2-(Benzhydryl-amino)-N-benzyl-2-phenyl-acetamide hydrochloride

Colorless solid: m.p. = 218.4-219.2°C (dec.); HPLC: t_{R} = 6.13 min (gradient C), ESI+ MS: m/z = 407.1 (MH⁺).

### Example 120: rac- 2-(Benzhydryl-amino)-N-(3-chloro-benzyl)-2-phenyl-acetamide hydrochloride

Colorless solid: m.p. = 215-216°C, HPLC: t_{R} = 6.54 min (gradient C), ESI+ MS: m/z = 441.2 (MH⁺).

### Example 121: rac-2-(Benzooxazo-2-ylamino)-N-benzyl-2-(4-fluoro-phenyl)-acetamide trifluoroacetate

a) A DMA solution (1 ml) containing (9H-Fluoren-9-ylmethoxycarbonylamino)-(4-fluorophenyl)-acetic acid (55 mg, 0.14 mmol, 2 eq.), HATU (53 mg, 0.14 mmol, 2 eq.), and DIPEA (48 µl, 0.28 mmol, 4 eq.) is prepared and added to benzylamine-BAL resin (85 mg, 70 µmol, loading = 0.8 mmol/g, LCC Reactospheres, LCC Engineering & Trading GmbH, CH-4622 Egerkingen, Switzerland) after 5 min of activation. The suspension is shaken overnight at room temperature. The solution is drained and fresh coupling solution (1 ml) is added and the mixture is shaken for further 3 hours. The resin is drained and washed with DMA (3x).
b) The resin obtained in step a) is suspended in 20% piperidine in DMA (1 ml) and shaken for 30 min. at room temperature. This procedure is repeated twice with fresh 20% piperidine in DMA (2x 1 ml). Then, the resin is drained and washed with DMA (3x), isopropanol (2x), DCE (2x), NMP (2x).
c) A NMP solution (0.5 ml) containing 2-chlorobenzooxazole (107 mg, 0.7 mmol, 5 eq.) and triethylamine (97 µl ,0.7 mmol, 5 eq.) is prepared and added to the resin obtained in step b). The suspension is shaken overnight at 95°C and the resin is washed with DMA (2x), DMA / H₂O 7:3 (1x), isopropanol (2x), DCE (2x). Crude product is obtained after cleavage in 20% TFA in DCM for 3.5 hours at room temperature, and purified by preparative HPLC (gradient G). Lyophilisation with *tert*.-butanol gives the title compound as colorless powder. ¹H-NMR (400 MHz, DMSO-d₆): δ 4.25 (dd, *J*=15.3, 5.5 Hz, 1H), 4.35 (dd, 1H), 5.53 (d, *J*=7.9 Hz, 1H), 7.02 (t, *J*=7.3 Hz, 1H), 7.14 (t, 1H), 7.24 (m, 8H), 7.38 (d, *J*=7.9 Hz, 1H), 7.59 (dd, *J*=9.2, 5.5 Hz, 2H), 8.79 (d, *J*=8.6 Hz, 1H), 8.94 (t, *J*=6.1 Hz, 1 H); HPLC: t_{R} = 5.5 min (gradient D), ESI+ MS: m/z = 376.4 (MH⁺).

### Examples 122 - 131

The following examples are prepared according to the procedures as described in Example 121.

### Example 122: rac-2-(Benzooxazol-2-ylamino)-2-(4-fluoro-phenyl)-N-methylacetamide trifluoro acetate

HPLC: t_{R} = 4.0 min (gradient D), ESI+ MS: m/z = 300.3 (MH⁺).

### Example 123: rac-N-Allyl-2-(benzooxazol-2-ylamino)-2-(4-fluoro-phenyl)-acetamide trifluoro acetate

HPLC: t_{R} = 4.7 min (gradient D), ESI+ MS: m/z = 326.3 (MH⁺).

### Example 124: rac-2-(Benzooxazol-2-ylamino)-2-(4-fluoro-phenyl)-N-(2-trifluoromethoxy-benzyl)-acetamide trifluoro acetate

HPLC: t_{R} = 6.3 min (gradient D), ESI+ MS: m/z = 460.4 (MH⁺).

### Example 125: rac-2-(5-Cyano-pyridin-2-ylamino)-N-cyclohexylmethyl-2-phenyl-acetamide trifluoro acetate

HPLC: t_{R} = 6.1 min (gradient D), ESI+ MS: m/z = 349.4 (MH⁺).

### Example 126: rac-2-(5-Cyano-pyridin-2-ylamino)-2-phenyl-N-(1-pyrimidin-2-yl piperidin-4-yl)-acetamide trifluoro acetate

HPLC: t_{R} = 3.9 min (gradient D), ESI+ MS: m/z = 414.4 (MH⁺).

### Example 127: rac-2-(5-Cyano-pyridin-2-ylamino)-N-(3-methyl-butyl)-2-phenyl-acetamide trifluoro acetate

HPLC: t_{R} = 5.6 min (gradient D), ESI+ MS: m/z = 323.4 (MH⁺).

### Example 128: rac- 2-(5-Cyano-pyridin-2-ylamino)-N-phenethyl-2-phenyl-acetamide trifluoro acetate

HPLC: t_{R} = 5.6 min (gradient D), ESI+ MS: m/z = 357.4 (MH⁺).

### Example 129: rac- 2-(5-Cyano-pyridin-2-ylamino)-N-(3,4-dimethoxy-benzyl)-2-phenyl-acet-amide trifluoroacetate

HPLC: t_{R} = 5.9 min (gradient D), ESI+ MS: m/z = 403.5 (MH⁺).

### Example 130: rac-2-(5-Cyano-pyridin-2-ylamino)-N-(2,3-dihydro-benzo[1,4]dioxin-2-ylmethyl)-2-phenyl-acetamide trifluoro acetate

HPLC: t_{R} = 5.7 min (gradient D), ESI+ MS: m/z = 401.4 (MH⁺).

### Example 131: rac-N-(2,2-Diphenyl-ethyl)-2-(4-fluoro-phenyl)-2-(5-methyl-thieno[2,3-d]-pyrimidin-4-ylamino)-acetamide hydrochloride

HPLC: t_{R} = 4.63 min (gradient E), ESI+ MS: m/z = 497.7 (MH⁺).

### Example 132: rac-2-Acetylamino-N-benzyl-2-phenyl-acetamide trifluoroacetate

To 2-amino-N-benzyl-N-BAL-2-phenyl-acetamide resin (160 mg, 0.128 mmol, loading 0.8 mmol g⁻¹, prepared according to example 4a, steps a) & b)) is added a mixture of acetic anhydride, pyridine and DMA 1:1:8 (1.2 ml). The suspension is shaken for 10 min at room temperature. The solution is drained, fresh acylation solution (1.2 ml) is added and the suspension is shaken for further 10 min. The resin is then drained and washed with DMA, methanol, water, DCM, methanol, and DCM (2x). Crude product is obtained after cleavage in 20% TFA in DCM (2x 1.5 hours), and purified by preparative HPLC (gradient G). Lyophilisation with *tert*.-butanol gives the title compound as colorless powder. HPLC: t_{R} = 4.0 min (gradient D), ESI+ MS: m/z = 283.1 (MH⁺).

### Examples 133 - 134

The following examples are prepared according to the procedures as described in Example 132.

### Example 133: rac-2-Acetylamino-N-(2-phenoxy-ethyl)-2-phenyl-acetamide trifluoro acetate

HPLC: t_{R} = 4.3 min (gradient D), ESI+ MS: m/z = 313.5 (MH⁺).

### Example 134: rac-2-Acetylamino-2-phenyl-N-(2-trifluoromethoxy-benzyl)-acetamide trifluoro acetate

HPLC: t_{R} = 5.1 min (gradient D), ESI+ MS: m/z = 367.5 (MH⁺).

### Example 135: rac-N-Benzyl-3-(4-chloro-benzylamino)-3-phenyl-proplonamide fumarate

The title compound is prepared by the procedure described in Example 99 starting from 3-*tert*-butoxycarbonylamino-3-phenyl-propionic acid: Colorless crystals, ¹H-NMR (400MHz; DMSO-d₆): δ 2.5 (ddd, 2H); 3.45 (ddd, 2H); 3.95 (t, 1H); 4.28 (ddd , 2H); 7.0-7.4 (m, 14H); 8.35 (t, 1H), ESI+ MS: m/z = 379.2 (MH⁺),

### Example 136: rac-N-(2,2-Diphenyl-ethyl)-3-phenyl-2-[(pyridin-2-ylmethyl)-amino]-propion-amide

The title compound is prepared in the same manner as described in Example 99 starting from 2-*tert-*butoxycarbonylamino-3-phenyl-propionic acid, 2,2-diphenyl-ethylamine and pyridine-2-carbaldehyde: Colorless crystals, mp. 68-70°C, ¹H-NMR (400MHz; CDCl₃): δ 2.65 (dd, 1 H); 3.1 (dd, 1 H) ;3.2 (dd, 1 H); 3.75 (m, 2H); 3.8-4.1 (abx, 2H); 4.2 (t , 1H); 6.85 (t, NH); 7.0-7.4 (m, 15H); 7.8 (t, 1 H); 8.6 (d, 1 H) ESI+ MS: m/z = 436.2 (MH⁺).

### Example 137: 10,11-Dihydro-dibenzo[b,f]azepine-5-carboxylic acid benzylamide

To a solution of 10,11-Dihydro-dibenzo[b,f]azepine-5-carbonyl chloride (194 mg, 0.602 mmol, CAS 33948-19-5) in DCM (2 ml) is added benzylamine (145 µl, 1.32 mmol) and the mixture is allowed to stir at room temperature for 27 hours. The mixture is concentrated *in vacuo* and purified by chromatography on silica gel (hexane / ethyl acetate gradient 85:15 - 0:100). The title compound is obtained as a white solid. HPLC: t_{R} = 3.16 (gradient c), ¹H-NMR (400MHz; CDCl₃): δ 2.70-2.90 (b, 2H), 3.30-3.50 (b, 2H), 4.44 (d, J=7 Hz, 2H), 4.89 (t, J=7 Hz, 1H); ESI+ m/z = 329.2 (MH⁺).

### Example 138: N,N'-Dibenzhydryl-ethane-1,2-diamine dihydro chloride

The synthesis of the title compound is described in EP058373A1 (Ciba-Geigy AG). Colorless crystals: mp 282-283°C.

### Example 139: N,N'-Bis-[bis-(4-fluoro-phenyl)-methyl]-ethane-1,2-diamine dihydro chloride

The synthesis of the title compound is described in EP058373A1 (Ciba-Geigy AG). Colorless crystals: mp 297-298°C.

### Example 140: N,N'-Bis-[(4-methoxy-phenyl)-phenyl-methyl]-ethane-1,2-diamine

The synthesis of the title compound is described in EP058373A1 (Ciba-Geigy AG).
Colorless crystals: mp 97-98°C.

### Example 141: N,N'-Bis-[(4-chloro-phenyl)-phenyl-methyl]-ethane-1,2-diamine dimesylate

(4-Chloro-phenyl)-phenyl-methanone (21.5 g, 99 mmol), ethane-1,2-diamine (25ml) are stirred for 20 hours at 180 °C. A suspension of sodium borohydride (13 g) in water (30 ml) is added to a solution of the crude compound dissolved in methanol (300 ml). The mixture is refluxed for 1 hour. Water is added and the mixture is extracted with methylenechloride, dried and evaporated to afford 23 g of crude compound. Methanesulfonic acid is added to a solution of the crude compound in acetone. The resulting crystals are the dimethane-sulfonate salt, mp 233-235°C, ESI+ MS: m/z = 463 (MH⁺).

### Example 142: (S,S)-N,N'-Di-chroman-4-yl-ethane-1,2-diamine di-(trifluoroacetate)

To an ice cooled solution of (*S*)-4-aminochromane (200 mg, 1.34 mmol) and triethyl-amine (207 µl, 1.47 mmol) in dry dichloro methane (5 ml) is added slowly oxalyl chloride (57 µl, 0.67 mmol) via a hypodermic syringe. Then, the light brown suspension is stirred for 3h at room temperature under argon. After this, the reaction mixture is poured on ice water. The precipitate is filtered off, washed with cold water followed by diethyl ether. The residue is vacuum dried at 50°C over night to give (*S,S*)-*N,N'*-di-chroman-4-yl-oxalamide as tan powder.
To a suspension of (*S,S*)-*N,N'*-di-chroman-4-yl-oxalamide (102 mg, 0.29 mmol) in dry THF (5 ml) is added slowly 1 M borane-THF complex in THF (4.92 ml, 4.63 mmol) via a hypodermic syringe under argon. After the effervescence has ceased the now colorless thick suspension is brought to reflux and kept at this temperature over night. After reaching room temperature the colorless clear reaction mixture is poured on a mixture of ethyl acetate (25 ml) and water (25 ml). The organic layer is separated and the water phase extracted twice with ethyl acetate (10 ml). The combined organic extracts are washed with brine, dried over Na₂SO₄ and evaporated to dryness. The residue is purified by preparative HPLC (gradient F) to afford the title compound as a colorless powder, HPLC: t_{R} = 3.35 min (gradient A); ESI+ MS:
m/z = 295 (MH⁺).

### Example 143: N-Benzhydryl-N'-(9H-fluoren-9-yl)-ethane-1,2-diamine dihydro chloride

N'1'-Benzhydryl-ethane-1,2-diamine (600 mg, 2 mmol) [prepared according to EP58373A1, Ciba-Geigy AG] is suspended in 10 mL DCM. To this mixture 1.4 mL triethylamine and 9-bromofluorene (540 mg, 2.2 mmol) are added and the reaction is stirred for 22 hours at room temperature. Subsequently DCM is added and the organic phase is washed with conc. NaHCO₃ solution. The water phase is re-extracted with DCM. The combined organic phases are dried over Na₂SO₄, filtered and evaporated to dryness. The residue is purified by flash chromatography (cyclohexane / ethyl acetate, 1:1) to yield the title compound. Colorless crystals: mp. 89.0 - 91.1 °C, ESI+ MS: m/z = 391 (MH⁺).

### Example 144: N-Benzhydryl-N'-(10,11-dihydro-5H-dibenzo[a,d]cyclohepten-5-yl)-ethane-1,2-diamine

Prepared in accordance to the procedures described in Example 143. Colorless resin , HPLC: t_{R} = 6.55 min (gradient Acetonitrile + 0.1% AcOH / water + 0.1 % AcOH, Acetonitrile from 0 to 100% in 8min. 100% acetonitril for 2min.; column :Waters XTerra® MS C 18, 4.6 x 100mm), ESI+ MS: m/z = 419 (MH⁺).

### Example 145: Benzyl-[2-(10,11-dihydro-dibenzo[b,f]azepin-5-yl)-ethyl]-amine

Synthesis according to CH368493, Geigy AG, 24.06.1958. ESI+ MS: m/z = 329 (MH⁺).

### Example 146: rac-[2-(10,11-Dihydro-dibenzo[b,f]azepin-5-yl)-propyl]-dimethyl-amine hydrochloride

Synthesis according to CH368493, Geigy AG, 24.06.1958. mp. 172 - 173°C.

### Example 147: 5-(2-Piperidin-1-yl-ethyl)-10,11-dihydro-5H-dibenzo[b,f]azepine hydro chloride

Synthesis according to W. Schindler, Helv.Chim.Acta; 37,472, 481,(1954); mp. 278 - 280°C.

### Example 148: Soft Capsules

5000 soft gelatin capsules, each comprising as active ingredient 0.05 g of one of the compounds of formula I mentioned in the preceding Examples, are prepared as follows:

### Composition

| | |
|---|---|
| Active ingredient | 250 g |
| Lauroglycol | 2 litres |

Preparation process: The pulverized active ingredient is suspended in Lauroglykol® (propylene glycol laurate, Gattefossé S.A., Saint Priest, France) and ground in a wet pulverizer to produce a particle size of about 1 to 3 µm. 0.419 g portions of the mixture are then introduced into soft gelatin capsules using a capsule-filling machine.

## Claims

1. Use of a diamine of formula I 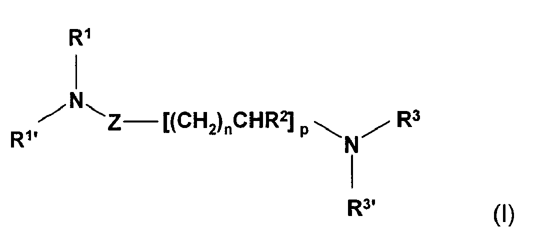 wherein
Z is -C(O)-, -C(S)- or a bond;
n is 0, 1, 2 or 3;
p is 1 or 0, under the proviso that p is 1 when Z is a bond;
R¹ denotes alkyl which is unsubstituted or mono-, di- or trisubstituted by alkoxy, hydroxy, halogen, amino, alkyl amino, di-alkyl amino, unsubstituted or substituted (C₄-C₁₂)aryloxy, unsubstituted or substituted (C₅-C₁₂)heterocyclyl containing oxygen, sulfur or nitrogen, unsubstituted or substituted (C₃-C₈)cycloalkyl or unsubstituted or substituted (C₄-C₁₄)aryl; alkenyl, which is unsubstituted or mono-, di- or trisubstituted by alkoxy, hydroxy, halogen, amino, alkyl amino, di-alkyl amino, unsubstituted or substituted (C₄-C₁₂)aryloxy, unsubstituted or substituted (C₅-C₁₂)heterocyclyl containing oxygen, sulfur or nitrogen, unsubstituted or substituted (C₃-C₈)cycloalkyl or unsubstituted or substituted (C₄-C₁₂)aryl; unsubstituted or substituted (C₃-C₈)cycloalkyl, unsubstituted or substituted (C₅-C₁₂)heterocyclyl containing oxygen, sulfur or nitrogen, unsubstituted or substituted (C₄-C₁₂)aryl or unsubstituted or substituted (C₄-C₁₂)hetaryl; and
R^{1'} denotes hydrogen, unsubstituted or substituted (C₄-C₁₂)aryl, unsubstituted or substituted (C₄-C₁₂)aryl alkyl or alkyl which is unsubstituted or mono-, di- or trisubstituted by alkoxy, hydroxy, halogen, amino, alkyl amino or di-alkyl amino; or
R¹ and R^{1'} together with the nitrogen atom to which they are attached form an unsubstituted or substituted mono-, di, tri or tetracyclic (C₅-C₁₆)heterocyclyl group containing at least one nitrogen atom,
R² denotes hydrogen or alkyl, unsubstituted or substituted (C₄-C₁₂)aryl alkyl or unsubstituted or substituted (C₄-C₁₂)aryl,
R³ denotes hydrogen, unsubstituted or substituted (C₄-C₁₆)aryl, unsubstituted or substituted (C₃-C₅)cycloalkyl, unsubstituted or substituted (C₅-C₁₂)heterocyclyl containing oxygen, sulfur or nitrogen; unsubstituted or substituted (C₄-C₁₂)hetaryl; alkyl which is unsubstituted or substituted by hydroxy, amino, unsubstituted or substituted (C₄-C₁₂)aryl, unsubstituted or substituted (C₄-C₁₂)hetaryl or unsubstituted or substituted aryl; or R¹⁷-C(O)-, wherein R¹⁷ denotes unsubstituted or substituted alkyl, unsubstituted or substituted (C₄-C₁₂)aryl or unsubstituted or substituted (C₄-C₁₂)hetaryl; and
R^{3'} denotes hydrogen, alkyl, alkyl carbonyl or unsubstituted or substituted (C₄-C₁₂)aryl alkyl, or
R³ and R^{3'} together with the nitrogen atom to which they are attached form an unsubstituted or substituted cyclic (C₅-C₁₂)heterocyclyl group containing at least one nitrogen atom,
for the manufacture of a medicament for the treatment or prevention of a disorder or condition in which the mGluR7 receptor plays a role or is implicated.

2. The use according to claim 1, wherein the disorder or condition in which the mGluR7 receptor plays a role or is implicated is selected from epilepsy, cerebral ischemia, eye disorders, especially glaucoma and ischemic diseases of the eye, itch, muscle spasms, convulsions or pain.

3. The use according to claim 1, wherein the disorder or condition in which the mGluR7 receptor plays a role or is implicated is selected from acute, traumatic and chronic degenerative processes of the nervous system and psychiatric diseases.

4. The use according to claim 3, wherein the disorder or condition is selected from Parkinson's disease, senile dementia, Alzheimer's disease, Huntington's chorea, amyotrophic lateral sclerosis and multiple sclerosis, schizophrenia, anxiety disorders and depression.

5. A method of treating a disorder or condition in which the mGluR7 receptor plays a role or is implicated, which method comprises administering to a subject in need of such treatment a therapeutically effective amount of a diamine of formula I as defined in claim 1 in free base or pharmaceutically acceptable acid addition salt form.

6. A diamine of formula I 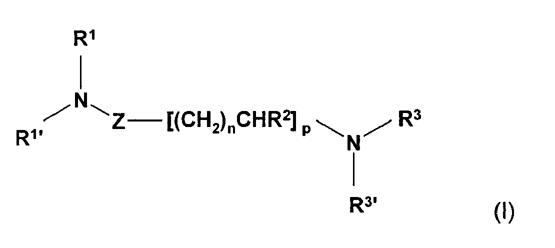 wherein
Z is -C(O)-, -C(S)- or a bond;
n is 0, 1, 2 or 3;
p is 1 or 0, under the proviso that p is 1 when Z is a bond;
R¹ denotes alkyl which is unsubstituted or mono-, di- or trisubstituted by alkoxy, hydroxy, halogen, amino, alkyl amino, di-alkyl amino, unsubstituted or substituted (C₄-C₁₂)aryloxy, unsubstituted or substituted (C₅-C₁₂)heterocyclyl containing oxygen, sulfur or nitrogen, unsubstituted or substituted (C₃-C₈)cycloalkyl or unsubstituted or substituted (C₄-C₁₄)aryl; alkenyl, which is unsubstituted or mono-, di- or trisubstituted by alkoxy, hydroxy, halogen, amino, alkyl amino, di-alkyl amino, unsubstituted or substituted (C₄-C₁₂)aryloxy, unsubstituted or substituted (C₅-C₁₂)heterocyclyl containing oxygen, sulfur or nitrogen, unsubstituted or substituted (C₃-C₈)cycloalkyl or unsubstituted or substituted (C₄-C₁₂)aryl; unsubstituted or substituted (C₃-C₈)cycloalkyl, unsubstituted or substituted (C₅-C₁₂)heterocyclyl containing oxygen, sulfur or nitrogen, unsubstituted or substituted (C₄-C₁₂)aryl or unsubstituted or substituted (C₄-C₁₂)hetaryl; and
R^{1'} denotes hydrogen, unsubstituted or substituted (C₄-C₁₂)aryl, unsubstituted or substituted (C₄-C₁₂)aryl alkyl or alkyl which is unsubstituted or mono-, di- or trisubstituted by alkoxy, hydroxy, halogen, amino, alkyl amino or di-alkyl amino; or
R¹ and R^{1'} together with the nitrogen atom to which they are attached form an unsubstituted or substituted mono-, di, tri or tetracyclic (C₅-C₁₆)heterocyclyl group containing at least one nitrogen atom,
R² denotes hydrogen or alkyl, unsubstituted or substituted (C₄-C₁₂)aryl alkyl or unsubstituted or substituted (C₄-C₁₂)aryl,
R³ denotes hydrogen, unsubstituted or substituted (C₄-C₁₆)aryl, unsubstituted or substituted (C₃-C₅)cycloalkyl, (C₅-C₁₂)heterocyclyl containing oxygen, sulfur or nitrogen; unsubstituted or substituted (C₄-C₁₂)hetaryl; alkyl which is unsubstituted or substituted by hydroxy, amino, (C₄-C₁₂)hetaryl or unsubstituted or substituted (C₄-C₁₂)aryl; or R¹⁷-C(O)-, wherein R¹⁷ denotes alkyl, (C₄-C₁₂)aryl or (C₄-C₁₂)hetaryl; and
R^{3'} denotes hydrogen, alkyl, alkyl carbonyl or unsubstituted or substituted (C₄-C₁₂)aryl alkyl, or
R³ and R^{3'} together with the nitrogen atom to which they are attached form an unsubstituted or substituted cyclic (C₅-C₁₂)heterocyclyl group containing at least one nitrogen atom,
under the proviso that if n is 0, Z is -C(O)-, p is 1, R¹ is benzyl, R² is phenyl and R¹' and R³' are hydrogen, then R³ does not denote benzyl or acetyl; if n is 0, Z is -C(O)-, p is 1, R¹ is phenethyl, R¹' is hydrogen, R² is phenyl and R³' is hydrogen, then R³ does not denote hydrogen; if n is 1, Z is a bond, p is 1, R¹ is di-phenyl methyl and R¹', R² and R³' are all hydrogen, then R³ is not di-phenyl methyl or benzyl; and, if Z is a bond, p is 1, R¹ and R¹' together represent the structure of subformula Ic, X represents -CH₂-CH₂-, R² is hydrogen and n is 1 or 2, then R³ and R³' together with the nitrogen atom to which they are attached do not represent piperidinyl and R³ and R³' are not simultaneously methyl; in free base or acid addition salt form.

7. A diamine of formula 1 according to claim 6
wherein
Z is -C(O)- or a bond;
n is 0, 1 or 2;
p is 1 or 0, under the proviso that p is 1 when Z is a bond;
R¹ denotes (C₁₋₆)alkyl which is unsubstituted or mono-, di- or trisubstituted by (C₁₋₆)alkoxy, hydroxy, halogen, amino, (C₁₋₆)alkyl amino, di-(C₁₋₆)alkyl amino, aryloxy, 2,3-dihydro-benzo[1,4]dioxanyl, (C₄-C₇)cycloalkyl or phenyl, which itself is unsubstituted or substituted by halogen, (C₁₋₆)alkoxy or trifluoromethoxy; (C₁₋₆)alkenyl, which is unsubstituted or mono-, di- or trisubstituted by (C₁₋₆)alkoxy, hydroxy, halogen, amino, (C₁₋₆)alkyl amino, di-(C₁₋₆)alkyl amino, aryloxy, 2,3-dihydro-benzo[1,4]dioxanyl, (C₄-C₇)cycloalkyl or phenyl, which itself is unsubstituted or substituted by halogen, (C₁₋₆)alkoxy or trifluoromethoxy; (C₄-C₇)cycloalkyl, 1,4-dioxan, naphthyl, 9H-fluorenyl, 10,11-dihydro-5H-dibenzo[a,d]cycloheptenyl, 2,3-dihydrobenzo[b]furyl, 2,3-dihydrochromenyl; phenyl which is unsubstituted or substituted by phenyl or (C₁₋₆)alkoxy; or piperidinyl which is unsubstituted or substituted by phenyl or pyrimidyl; and
R^{1'} denotes hydrogen, phenyl, benzyl or (C₁₋₆)alkyl which is unsubstituted or mono-, di- or trisubstituted by (C₁₋₆)alkoxy, hydroxy, halogen, amino, (C₁₋₆)alkyl amino or di-(C₁₋₆)alkyl amino; or
R¹ and R^{1'} together with the nitrogen atom to which they are attached form a cyclic structure of formula Ia, Ib, Ic or Id, 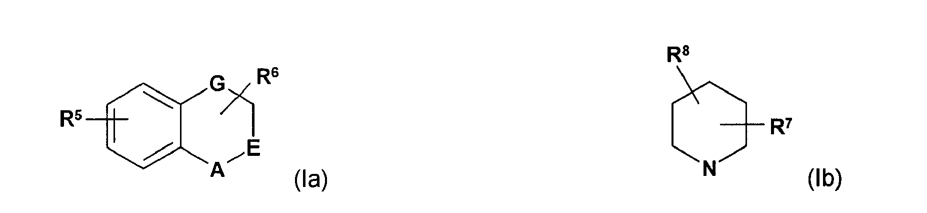 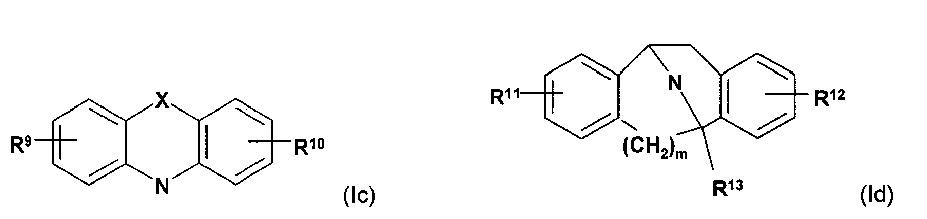 wherein
A denotes N and E denotes CR⁴R⁴' or A denotes CR⁴R⁴' and E denotes N,
G denotes a single bond, CHR¹⁹, -CH₂CH₂-, -CH=CH-, S(O)ₜor NR²⁰, wherein t is 0, 1 or 2, and R¹⁹ and R²⁰, independently of each other, represent hydrogen or (C₁₋₆)alkyl, which is unsubstituted or mono-, di- or trisubstituted by (C₁₋₆)alkoxy, hydroxy, halogen, amino, (C₁₋₆)alkyl amino or di-(C₁₋₆)alkyl amino
X represents oxygen, a single bond, CHR²¹, -CH=CH- or -CH₂-CH₂-, S(O)ᵣ or NR²²,
wherein r is 0, 1 or 2, and R²¹ and R²², independently of each other, represent hydrogen or (C₁₋₆)alkyl, which is unsubstituted or mono-, di- or trisubstituted by (C₁₋₆)alkoxy, hydroxy, halogen, amino, (C₁₋₆)alkyl amino or di-(C₁₋₆)alkyl amino,
m is 0 or 1,
R⁴ and R⁴' represent, independently of each other, hydrogen or (C₁₋₆)alkyl, which is unsubstituted or mono-, di- or trisubstituted by (C₁₋₆)alkoxy, hydroxy, halogen, amino, (C₁₋₆)alkyl amino or di-(C₁₋₆)alkyl amino,
R⁵ represents hydrogen, halogen, (C₁₋₆)alkoxy, phenyl or (C₁₋₆)alkyl which is unsubstituted or mono-, di- or trisubstituted by (C₁₋₆)alkoxy, hydroxy, halogen, amino, (C₁₋₆)alkyl amino or di-(C₁₋₆)alkyl amino,
R⁶ represents hydrogen, halogen, phenyl or (C₁₋₆)alkyl which is unsubstituted or mono-, di- or trisubstituted by (C₁₋₆)alkoxy, hydroxy, halogen, amino, (C₁₋₆)alkyl amino or di-(C₁₋₆)alkyl amino,
R⁷ and R⁸ represent, independently of each other, hydrogen, halogen or (C₁₋₆)alkyl, which is unsubstituted or mono-, di- or trisubstituted by (C₁₋₆)alkoxy, hydroxy, halogen, amino, (C₁₋₆)alkyl amino or di-(C₁₋₆)alkyl amino,
R⁹ and R¹⁰ represent, independently of each other, hydrogen, halogen, (C₁₋₆)alkoxy, phenyl or (C₁₋₆)alkyl which is unsubstituted or mono-, di- or trisubstituted by (C₁₋₆)alkoxy, hydroxy, halogen, amino, (C₁₋₆)alkyl amino or di-(C₁₋₆)alkyl amino,
R¹¹ is hydrogen, halogen, (C₁₋₆)alkoxy, phenyl or (C₁₋₆)alkyl which is unsubstituted or mono-, di- or trisubstituted by (C₁₋₆)alkoxy, hydroxy, halogen, amino, (C₁₋₆)alkyl amino or di-(C₁₋₆)alkyl amino,
R¹² is hydrogen, halogen, (C₁₋₆)alkoxy, phenyl or (C₁₋₆)alkyl which is unsubstituted or mono-, di- or trisubstituted by (C₁₋₆)alkoxy, hydroxy, halogen, amino, (C₁₋₆)alkyl amino or di-(C₁₋₆)alkyl amino, and
R¹³ represents hydrogen or (C₁₋₆)alkyl which is unsubstituted or mono-, di- or trisubstituted by (C₁₋₆)alkoxy, hydroxy, halogen, amino, (C₁₋₆)alkyl amino or di-(C₁₋₆)alkyl amino,
R² denotes hydrogen or (C₁₋₆)alkyl, or benzyl, naphthyl or phenyl, which in each case is unsubstituted or substituted by halogen, (C₁₋₆)alkoxy, (C₁₋₆)alkyl, hydroxy, trifluoromethyl, amino, (C₁₋₆)alkyl amino or di-(C₁₋₆)alkyl amino,
R³ denotes hydrogen, fluorenyl, 2,3-dihydrochromenyl, 1,2,2a,3,4,5-hexahydro-acenaphthyl, 1,2-dihydro-acenaphthyl, tetrahydronaphthyl which is unsubstituted or substituted by (C₁₋₆)alkoxy; benzoxazolyl; cyano-pyridyl; (C₁₋₆)alkyl thieno[2,3-d]-pyrimidyl; N-(C₁₋₆)alkyl piperidinyl, which is substituted by phenyl; (C₃-C₄)cycloalkyl which is substituted by phenyl; 2,3-dihydro-indenyl; (C₁₋₆)alkyl which is unsubstituted or substituted by hydroxy, amino, naphthyl, indolyl, thiazolyl, pyridyl or phenyl, which itself is unsubstituted or mono- or disubstituted by halogen, phenyl, nitro, di-(C₁₋₆)alkyl amino, di-(C₁₋₆)alkyl amino (C₁₋₆)alkoxy, (C₁₋₆)alkyl, (C₁₋₆)alkoxy, trifluoromethyl or trifluoromethoxy; or R¹⁷-C(O)-,
wherein R¹⁷ denotes (C₁₋₆)alkyl, phenyl, indolyl or benzo[b]furyl, which in each case is substituted by cyano, carboxy or (C₁₋₆)alkoxy carbonyl; and
R^{3'} denotes hydrogen, (C₁₋₆)alkyl, (C₁₋₆)alkyl carbonyl or benzyl, or
R³ and R^{3'} together with the nitrogen atom to which they are attached form a cyclic structure of formula Ie or If, 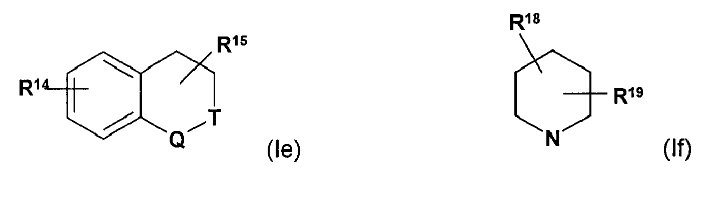 wherein
Q denotes N and T denotes CR¹⁶R¹⁶' or Q denotes CR¹⁶R¹⁶' and T denotes N,
R¹⁴ denotes hydrogen, halogen, (C₁₋₆)alkoxy, phenyl or (C₁₋₆)alkyl which is unsubstituted or mono-, di- or trisubstituted by (C₁₋₆)alkoxy, hydroxy, halogen, amino, (C₁₋₆)alkyl amino or di-(C₁₋₆)alkyl amino,
R¹⁵, R¹⁶, R^{16'} R¹⁷ and R¹⁸ represent, independently of each other, hydrogen, halogen or (C₁₋₆)alkyl, which is unsubstituted or mono-, di- or trisubstituted by (C₁₋₆)alkoxy, hydroxy, halogen, amino, (C₁₋₆)alkyl amino or di-(C₁₋₆)alkyl amino, under the proviso that if n is 0, Z is -C(O)-, p is 1, R¹ is benzyl, R² is phenyl and R¹' and R³' are hydrogen, then R³ does not denote benzyl or acetyl; if n is 0, Z is -C(O)-, p is 1, R¹ is phenethyl, R¹' is hydrogen, R² is phenyl and R³' is hydrogen, then R³ does not denote hydrogen; if n is 1, Z is a bond, p is 1, R¹ is di-phenyl methyl and R¹', R² and R³' are all hydrogen, then R³ is not di-phenyl methyl or benzyl; and, if Z is a bond, p is 1, R¹ and R¹' together represent the structure of subformula Ic, X represents -CH₂-CH₂-, R² is hydrogen and n is 1 or 2, then R³ and R³' together with the nitrogen atom to which they are attached do not represent piperidinyl and R³ and R³' are not simultaneously methyl; in free base or acid addition salt form. 8.

8. A diamine of formula I according to claim 6, wherein
Z is -C(O)- or a bond;
n is 0, 1 or 2;
p is 1 or 0, under the proviso that p is 1 when Z is a bond;
R¹ denotes (C₁₋₆)alkyl which is unsubstituted or mono- or disubstituted by phenyl, which itself is unsubstituted or substituted by halogen, (C₁₋₆)alkoxy or trifluoromethoxy, phenoxy, 2,3-dihydro-benzo[1,4]dioxanyl or (C₅-C₇)cycloalkyl; (C₁₋₆)alkenyl; naphthyl, 9H-fluorenyl, 10,11-dihydro-5H-dibenzo[a,d]cycloheptenyl, 2,3-dihydrobenzo[b]furyl, 2,3-dihydrochromenyl; phenyl which is unsubstituted or substituted by phenyl or (C₁₋₆)alkoxy; or piperidinyl which is substituted by pyrimidyl; and
R^{1'} denotes phenyl, hydrogen or (C₁₋₆)alkyl; or
R¹ and R^{1'} together with the nitrogen atom to which they are attached form a cyclic structure of formula Ia, Ib, Ic or Id, wherein A denotes N and E denotes CR⁴R⁴' or A denotes CR⁴R⁴' and E denotes N, G denotes CH₂, X represents oxygen, a bond, -CH=CH- or -CH₂-CH₂-, m is 0 or 1, R⁴ and R⁴' represent, independently of each other, hydrogen or (C₁₋₆)alkyl, R⁵ represents hydrogen, (C₁₋₆)alkyl, CF₃ or halogen, R⁶ represents hydrogen or (C₁₋₆)alkyl, R⁷ and R⁸ are both hydrogen, R⁹ and R¹⁰ are both hydrogen, R¹¹ is hydrogen or (C₁₋₆)alkoxy, R¹² represents hydrogen, and R¹³ represents hydrogen or (C₁₋₆)alkyl,
R² denotes hydrogen, (C₁₋₆)alkyl, benzyl, or phenyl, which is unsubstituted or substituted by halogen,
R³ denotes hydrogen, fluorenyl, 2,3-dihydrochromenyl, 1,2,2a,3,4,5-hexahydro-acenaphthyl, 1,2-dihydro-acenaphthyl, tetrahydronaphthyl which is unsubstituted or substituted by (C₁₋₆)alkoxy; benzoxazolyl; cyano-pyridyl; (C₁₋₆)alkyl thieno[2,3-d]-pyrimidyl; N-(C₁₋₆)alkyl piperidinyl, which is substituted by phenyl; (C₃-C₄)cycloalkyl which is substituted by phenyl; 2,3-dihydro-indenyl; (C₁₋₆)alkyl which is unsubstituted or substituted by hydroxy, amino, naphthyl, indolyl, thiazolyl, pyridyl or phenyl, which itself is unsubstituted or mono- or disubstituted by halogen, phenyl, nitro, di-(C₁₋₆)alkyl amino, di-(C₁₋₆)alkyl amino (C₁₋₆)alkoxy, (C₁₋₆)alkyl, (C₁₋₆)alkoxy, trifluoromethyl or trifluoromethoxy; or R¹⁷-C(O)-,
wherein R¹⁷ denotes (C₁₋₆)alkyl, phenyl, indolyl or benzo[b]furyl, which in each case is substituted by cyano, carboxy or (C₁₋₆)alkoxy carbonyl; and
R^{3'} denotes hydrogen, (C₁₋₆)alkyl, (C₁₋₆)alkyl carbonyl or benzyl, or
R³ and R^{3'} together with the nitrogen atom to which they are attached form a cyclic structure of formula Ie or If, wherein
Q denotes N and T denotes CR¹⁶R¹⁶' or Q denotes CR¹⁶R¹⁶' and T denotes N, and
R¹⁴, R¹⁵, R¹⁶, R^{16'} R¹⁷ and R¹⁸ are all hydrogen,
under the proviso that if n is 0, Z is -C(O)-, p is 1, R¹ is benzyl, R² is phenyl and R¹' and R³' are hydrogen, then R³ does not denote benzyl or acetyl; if n is 0, Z is -C(O)-, p is 1, R¹ is phenethyl, R¹_{,} is hydrogen, R² is phenyl and R³' is hydrogen, then R³ does not denote hydrogen; if n is 1, Z is a bond, p is 1, R¹ is di-phenyl methyl and R¹', R² and R³' are all hydrogen, then R³ is not di-phenyl methyl or benzyl; and, if Z is a bond, p is 1, R¹ and R¹' together represent the structure of subformula Ic, X represents -CH₂-CH₂-, R² is hydrogen and n is 1 or 2, then R³ and R³' together with the nitrogen atom to which they are attached do not represent piperidinyl and R³ and R³' are not simultaneously methyl; in free base or acid addition salt form.

9. A diamine of formula I according to claim 6 in free base or pharmaceutically acceptable acid addition salt form, for use as a pharmaceutical.

10. A pharmaceutical composition comprising a diamine of formula I according to claim 6 in free base or pharmaceutically acceptable acid addition salt form and at least one pharmaceutically acceptable carrier.

11. A process for the preparation of a diamine of formula I 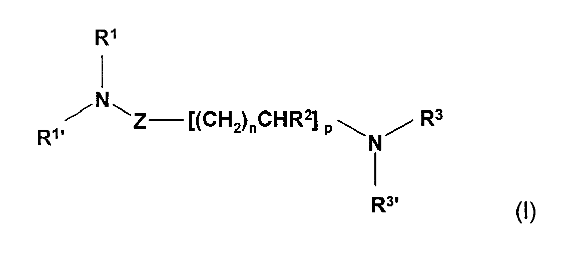 wherein
Z is -C(O)-, -C(S)- or a bond;
n is 0, 1, 2 or 3;
p is 1 or 0, under the proviso that p is 1 when Z is a bond;
R¹ denotes alkyl which is unsubstituted or mono-, di- or trisubstituted by alkoxy, hydroxy, halogen, amino, alkyl amino, di-alkyl amino, unsubstituted or substituted (C₄-C₁₂)aryloxy, unsubstituted or substituted (C₅-C₁₂)heterocyclyl containing oxygen, sulfur or nitrogen, unsubstituted or substituted (C₃-C₈)cycloalkyl or unsubstituted or substituted (C₄-C₁₄)aryl; alkenyl, which is unsubstituted or mono-, di- or trisubstituted by alkoxy, hydroxy, halogen, amino, alkyl amino, di-alkyl amino, unsubstituted or substituted (C₄-C₁₂)aryloxy, unsubstituted or substituted (C₅-C₁₂)heterocyclyl containing oxygen, sulfur or nitrogen, unsubstituted or substituted (C₃-C₈)cycloalkyl or unsubstituted or substituted (C₄-C₁₂)aryl; unsubstituted or substituted (C₃-C₈)cycloalkyl, unsubstituted or substituted (C₅-C₁₂)heterocyclyl containing oxygen, sulfur or nitrogen, unsubstituted or substituted (C₄-C₁₂)aryl or unsubstituted or substituted (C₄-C₁₂)hetaryl; and
R^{1'} denotes hydrogen, unsubstituted or substituted (C₄-C₁₂)aryl, unsubstituted or substituted (C₄-C₁₂)aryl alkyl or alkyl which is unsubstituted or mono-, di- or trisubstituted by alkoxy, hydroxy, halogen, amino, alkyl amino or di-alkyl amino; or
R¹ and R^{1'} together with the nitrogen atom to which they are attached form an unsubstituted or substituted mono-, di, tri or tetracyclic (C₅-C₁₆)heterocyclyl group containing at least one nitrogen atom,
R² denotes hydrogen or alkyl, unsubstituted or substituted (C₄-C₁₂)aryl alkyl or unsubstituted or substituted (C₄-C₁₂)aryl,
R³ denotes hydrogen, unsubstituted or substituted (C₄-C₁₆)aryl, unsubstituted or substituted (C₃-C₅)cycloalkyl, (C₅-C₁₂)heterocyclyl containing oxygen, sulfur or nitrogen; unsubstituted or substituted (C₄-C₁₂)hetaryl; alkyl which is unsubstituted or substituted by hydroxy, amino, (C₄-C₁₂)aryl, (C₄-C₁₂)hetaryl or unsubstituted or substituted aryl; or R¹⁷-C(O)-, wherein R¹⁷ denotes alkyl, (C₄-C₁₂)aryl or (C₄-C₁₂)hetaryl; and
R^{3'} denotes hydrogen, alkyl, alkyl carbonyl or unsubstituted or substituted (C₄-C₁₂)aryl alkyl, or
R³ and R^{3'} together with the nitrogen atom to which they are attached form an unsubstituted or substituted cyclic (C₅-C₁₂)heterocyclyl group containing at least one nitrogen atom,
under the proviso that if n is 0, Z is -C(O)-, p is 1, R¹ is benzyl, R² is phenyl and R¹' and R³' are hydrogen, then R³ does not denote benzyl or acetyl; if n is 0, Z is -C(O)-, p is 1, R¹ is phenethyl, R¹' is hydrogen, R² is phenyl and R³' is hydrogen, then R³ does not denote hydrogen; if n is 1, Z is a bond, p is 1, R¹ is di-phenyl methyl and R¹', R² and R³' are all hydrogen, then R³ is not di-phenyl methyl or benzyl; and, if Z is a bond, p is 1, R¹ and R¹' together represent the structure of subformula Ic, X represents -CH₂-CH₂-, R² is hydrogen and n is 1 or 2, then R³ and R³' together with the nitrogen atom to which they are attached do not represent piperidinyl and R³ and R³' are not simultaneously methyl;
(a) wherein Z is C(O) and the other radicals and symbols have the meanings as provided for a compound of formula I above comprising the step of reacting a halide of formula II 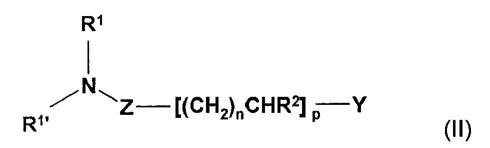 wherein n, p, Z, R¹, R^{1'} and R² are defined as for a compound of formula I above, and Y is Cl, Br or I with an amine of formula III 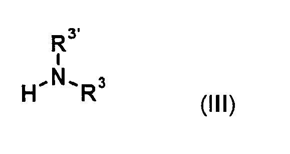 wherein R³ and R^{3'} are defined as for a compound of formula I above;
(b) wherein Z is C(O) and the other radicals and symbols have the meanings as provided for a compound of formula I above, is prepared by coupling a carboxylic acid of formula IV 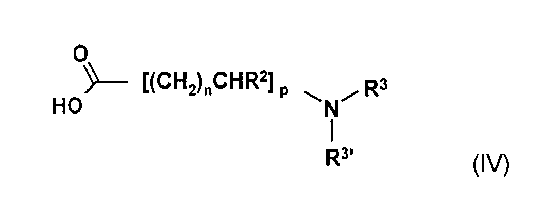 wherein n, p, R³, R^{3'} and R² are as defined above for a compound of formula I, with a primary or secondary amine of formula V 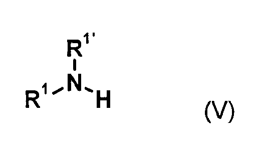 wherein R¹ and R^{1'} are as defined above for a compound of formula I;
(c) wherein Z is C(O), R³ is R¹⁷-C(O)-, wherein R¹⁷ denotes unsubstituted or substituted alkyl, or R³ is alkyl which is unsubstituted or substituted by hydroxy, amino, unsubstituted or substituted (C₄-C₁₂)aryl, unsubstituted or substituted (C₄-C₁₂)hetaryl or unsubstituted or substituted aryl, R³_{,} is hydrogen and the other radicals and symbols have the meanings as provided for a compound of formula I above, is synthesized by alkylation, reductive alkylation or acylation of the diamine of formula I, wherein R³ and R³' are both hydrogen, with an alkylating agent, a carbonyl compound or an acylating agent;
(d) wherein Z is C(O), p is 0 and the other radicals have the meanings as provided for a compound of formula I above, can be prepared by reacting a carbamoyl chloride of formula VI 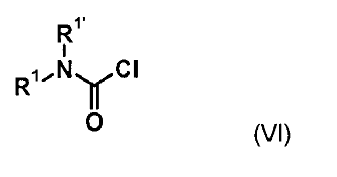 wherein R¹ and R^{1'} defined as above for a compound of formula I, with a primary or secondary amine of formula III 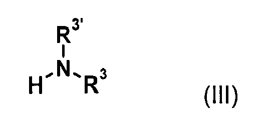 wherein R³ and R^{3'} are defined as above for a compound of formula I; or
(e) wherein Z is a bond and the other radicals have the meanings as provided for a compound of formula I above, is prepared by subjecting a diamine of formula VII 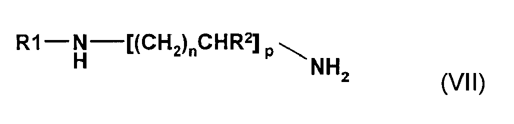
wherein n, p, R¹ and R² are as defined above for a compound of formula I, alkylation or acylation to provide diamines of formula I;
wherein the starting compounds of formula II to VII may also be present with functional groups in protected form, if necessary, and/or in the form of salts, provided a salt-forming group is present and the reaction in salt form is possible;
wherein any protecting groups in a protected derivative of a diamine of the formula I are removed; and, if so desired, a free diamine of formula I is converted into a salt, an obtainable salt of a diamine of formula I is converted into the free diamine or another salt, and/or a mixture of isomeric diamines of formula I is separated into the individual isomers.
